(19) Europäisches Patentamt
European Patent Office
Office européen des brevets

(11) Publication number: **0 204 148**
**A2**

(12) **EUROPEAN PATENT APPLICATION**

(21) Application number: **86105976.4**

(22) Date of filing: **30.04.86**

(51) Int. Cl.$^4$: **C 07 D 319/20**
C 07 D 265/36, C 07 D 405/08
C 07 D 413/08, C 07 D 417/08
A 61 K 31/535, A 61 K 31/335
A 61 K 31/54

(30) Priority: **07.05.85 GB 8511526**

(43) Date of publication of application:
**10.12.86 Bulletin 86/50**

(84) Designated Contracting States:
**AT BE CH DE FR IT LI LU NL SE**

(71) Applicant: **HOECHST U.K. LIMITED**
**Hoechst House Salisbury Road**
**Hounslow Middlesex TW4 6JH(GB)**

(72) Inventor: **Allen, Robert Manning**
**6 Dormans Close Milton Keynes Village**
**Milton Keynes Bucks., MK10 9AR(GB)**

(72) Inventor: **Fletcher, Stephen Robert**
**11 Bowles Place Woughton-on-the-Green**
**Milton Keynes Bucks., MK6 3BB(GB)**

(74) Representative: **Kockläuner, Reinhard, Dr. et al,**
**HOECHST AKTIENGESELLSCHAFT Zentrale**
**Patentabteilung Postfach 80 03 20**
**D-6230 Frankfurt am Main 80(DE)**

(54) **]1,4[Benzodioxin and ]1,4[benzoxazine derivatives.**

(57) A compound of formula I

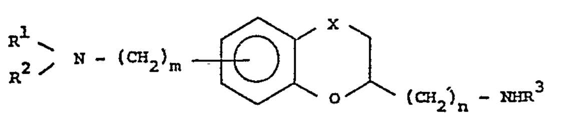

in which formula

$R^1$ and $R^2$, which may be the same or different, each represents a hydrogen atom, an alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl group having from 1 to 4 carbon atoms as substituent.

X represents an oxygen atom or a group $NR^4$ in which $R^4$ represents a hydrogen atom, an alkyl group having from 1 to 4 carbon atoms, a phenyl group or a phenalkyl group, the alkyl moiety of which has from 1 to 4 carbon atoms.

an alkyl, phenyl or phenalkyl group being unsubstituted or substituted, or

$R^4$ represents a nitrogen protecting group;

m represents an integer of from 1 to 4;

n represents the integer 1 or 2; and

$R^3$ represents a group of formula II, III, IV, V or VI

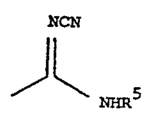

(II)

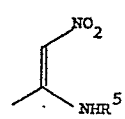

(III)

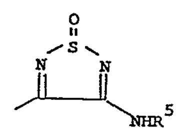

(IV)

(V)

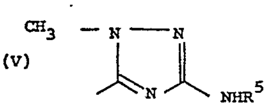

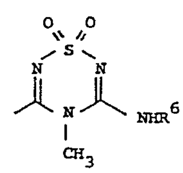

(VI)

in which formulae

$R^5$ represents a hydrogen atom or an alkyl group having from 1 to 4 carbon atoms; and $R^6$ represents a hydrogen atom or an alkyl group having from 1 to 4 carbon atoms, an alkenyl group having from 2 to 4 carbon atoms, or an alkynyl group having 3 or 4 carbon atoms has histamine H-2 antagonist activity, and may be used to produce medicaments.

EP 0 204 148 A2

# [1,4]BENZODIOXIN AND [1,4]BENZOXAZINE DERIVATIVES

The present invention relates to [1,4]benzodioxin and [1,4]benzoxazine derivatives which have histamine H-2 antagonist activity. The invention also relates to processes for the preparation of these derivatives and their salts, to pharmaceutical preparations comprising them, and to their use.

Histamine is one of a number of naturally occurring physiologically active substances which are thought to interact with specific receptors. In the case of histamine, there are at least two types: one is called the H-1 receptor (Ash and Schild, Brit. J. Pharmac. 1966, 27, 427), and the other is called the H-2 receptor (Black et al Nature 1972, 236, 385). The action of histamine at the H-1 receptor, for example, stimulation of bronchial and gastro-intestinal smooth muscle, is blocked by the compounds generally known as "antihistamines", but which are now also called histamine H-1 antagonists, for example, mepyramine. The action of histamine at the H-2 receptors, for example, stimulation of gastric acid secretion and heart rate, is not blocked by mepyramine but is blocked by other compounds, for example, burimamide and cimetidine.

Histamine H-2 antagonists may be used to treat those conditions resulting from stimulation by histamine of H-2 receptors, either alone, for example, in inhibiting gastric acid secretion and thus treating its sequelae, for example, gastric and peptic ulcers; or together with H-1 antagonists, for example, in allergic and certain inflammatory conditions.

The present invention provides compounds of formula I, which are histamine H-2 antagonists:

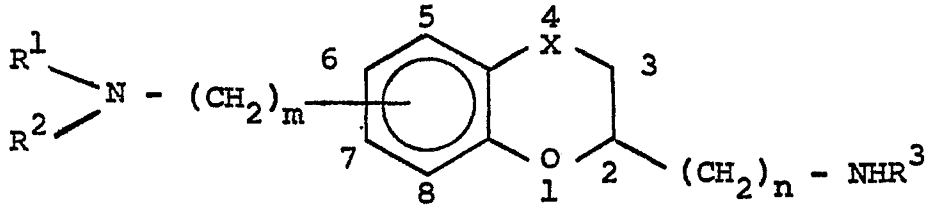

(I)

in which formula

$R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl group having from 1 to 4 carbon atoms as substituent,

X represents an oxygen atom or a group $NR^4$ in which $R^4$ represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substituents, which may be the same or different, for example, by one or two substituents, especially by one substituent, selected from halogen atoms, straight and branched chain alkyl groups having from 1 to 4 carbon atoms, straight and branched chain alkoxy groups having from 1 to 4 carbon atoms, nitro and trifluoromethyl groups,

or $R^4$ represents a nitrogen protecting group;

m represents an integer of from 1 to 4;

n represents 1 or 2; and

$R^3$ represents a group of formula II, III, IV, V or VI

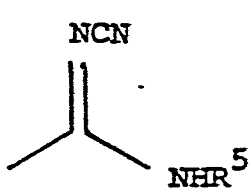

(II)

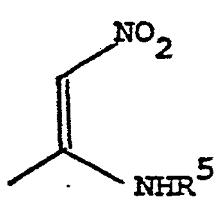

(III)

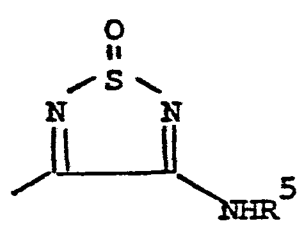

(IV)

(V) 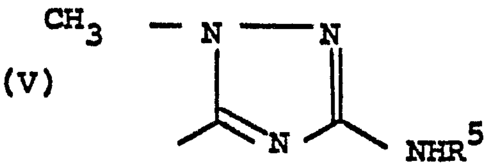

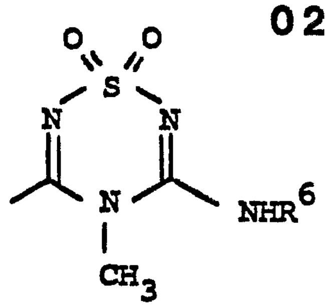 (VI)

in which formulae

$R^5$ represents a hydrogen atom or a straight or branched chain alkyl group having from 1 to 4 carbon atoms; and

$R^6$ represents a hydrogen atom or a straight or branched chain alkyl group having from 1 to 4 carbon atoms, a straight or branched chain alkenyl group having from 2 to 4 carbon atoms, or a straight or branched chain alkynyl group having 3 or 4 carbon atoms.

In a compound of formula I, when X represents an oxygen atom, $R^3$ preferably represents a group of formula V or VI, and in formula VI, $R^6$ preferably represents a hydrogen atom or a propargyl group. A preferred value for n is 2. Of particular interest are those compounds in which the group

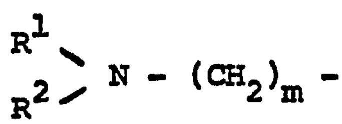

is in the 7-position, especially when n is 2 and $R^3$ preferably represents a group of formula V or VI as described above.

When X represents a group NH, n is preferably 2 and $R^3$ preferably represents a group of formula VI in which $R^6$ preferably represents a hydrogen atom. Again, the group

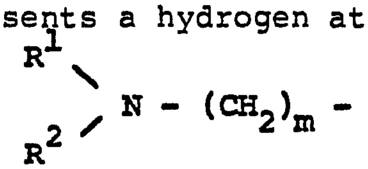

is preferably in the 7-position.

When $R^1$ and $R^2$ form a 4 to 8-membered ring which may contain an oxygen atom or another nitrogen atom, which nitrogen atom may have an alkyl group having from 1 to 4 carbon atoms as substituent, such a ring is, for example,

a pyrrolidine, piperidine, morpholine or N-methylpiperazine ring.

$R^4$ may represent a nitrogen protecting group. Such groups are well known in the art and are described, for example, in Protecting Groups in Organic Chemistry, McOmie, Plenum Press, London 1973, Chapter 2, pages 43 to 93, which are incorporated herein by reference, and in Protective Groups in Organic Synthesis, T.W. Greene, J. Wiley & Sons Inc. 1981, pages 218 to 287, which are also incorporated herein by reference. (It will be appreciated that some of the groups defined per se for $R^4$ may fall within the general definition of nitrogen protecting groups.)

Examples of nitrogen protecting groups are alkoxycarbonyl and aryloxycarbonyl groups, for example, p-nitrobenzyloxycarbonyl and t-butyloxycarbonyl groups; aralkyl groups, especially benzyl and substituted benzyl groups, for example, p-nitrobenzyl and p-methoxybenzyl groups; and alkylcarbonyl groups which may be substituted, for example, by halogen atoms, for example, trifluoromethylcarbonyl groups. Such groups may be removed, if desired, by conventional methods, for example, as described in McOmie and in Greene (see above). An example of a suitable method is acidic cleavage, for example, by treatment with hydrochloric acid.

The present invention also provides salts of compounds of formula I, especially physiologically tolerable salts, for example, salts with hydrochloric, sulphuric, hydrobromic, succinic, tartaric or maleic acid, also oxalate salts. The invention further provides hydrates, and solvates of formula I.

It will be understood that the structure given above for formula I is only one of several possible tautomeric and isomeric representations. The present invention includes all tautomeric and isomeric forms of compounds of the general formula I.

The term "lower" is used in the present specification

to denote a group, radical or molecule having up to 4 carbon atoms. Any alkyl group in the present specification may have a straight or branched chain.

The present invention also provides a process for the production of a compound of formula I, which comprises (i) reacting a compound of formula VII

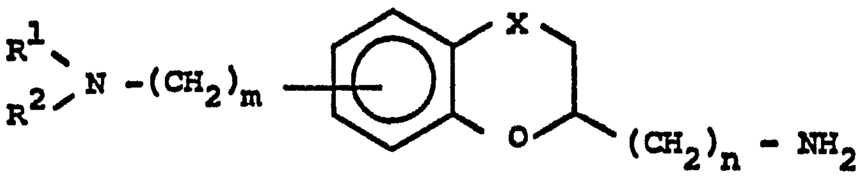

(VII)

in which $R^1$, $R^2$, X, m and n are as defined above, with a compound of formula IIa or IIIa

(IIa) 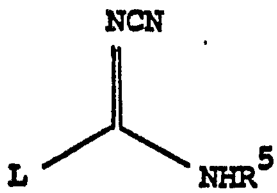 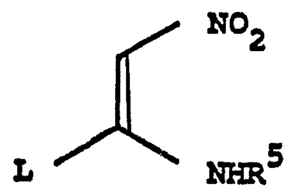 (IIIa)

in which L represents a leaving group, for example, a halogen atom or a group $-YR^7$ in which Y represents an oxygen or sulphur atom, a group SO or a group $SO_2$, and $R^7$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety, wherein a phenyl or phenalkyl group may be substituted by one or two, and especially by one substituent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms; and $R^5$ is as defined above, to obtain a compound of formula I in which $R^3$ represents a group of formula II or III respectively; or
(ii) reacting a compound of formula VII as defined above with a compound of formula VIII

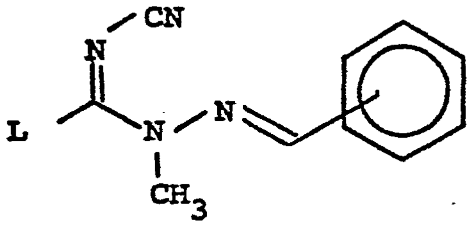

(VIII)

in which L is as defined above, and cyclising the resulting compound of formula IX

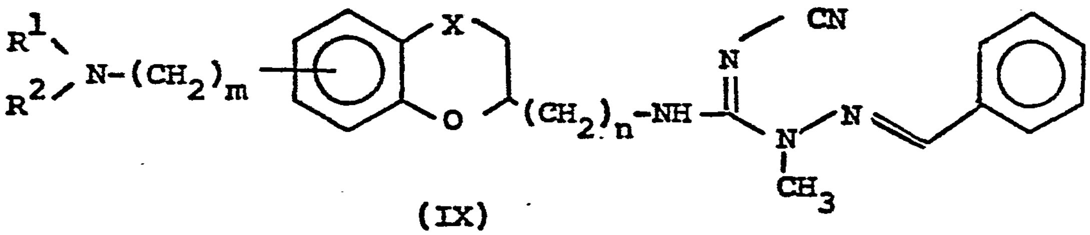

(IX)

to obtain a compound of formula I in which $R^3$ represents a group of formula V; or

(iii) reacting a compound of formula VII as defined above with a compound of formula IVa or VIa

(IVa)

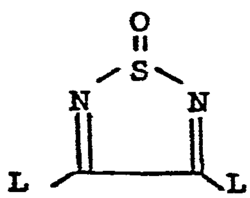

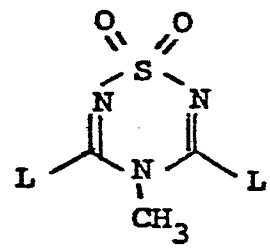

(VIa)

in which the two groups L each represents a leaving group as defined above, the two groups L being the same or different with the proviso that in the case of two leaving groups $-YR^7$ the two radicals $R^7$ can be the same or different, but the two groups Y must be the same, to give a compound of formula IVb or VIb respectively,

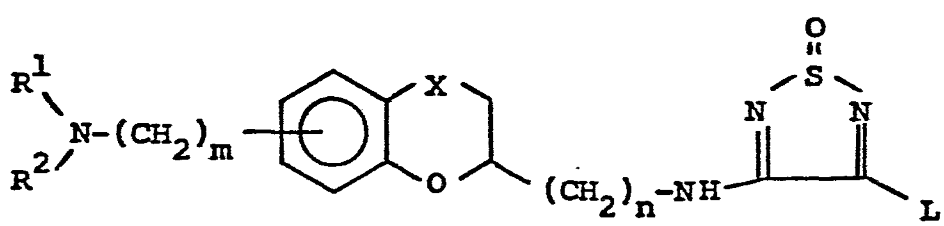

(IVb)

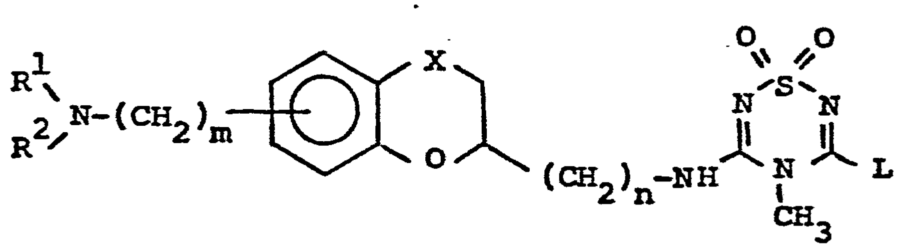

(VIb)

and reacting the resulting compound of formula IVb or VIb with a compound of formula Xa or Xb, respectively

(Xa) $H_2NR^5$ $H_2NR^6$ (Xb)

in which $R^5$ and $R^6$ are as defined above, to give a compound of formula I in which $R^3$ represents a group IV or VI, respectively.

A phenyl or phenalkyl group $R^7$ in a leaving group L may be substituted by one or more substituents, especially by one substituent, selected from straight and branched chain alkyl groups having from 1 to 4 carbon atoms, especially methyl groups, halogen atoms, straight and branched chain alkoxy groups having from 1 to 4 carbon atoms, especially methoxy groups, and nitro groups.

In a leaving group L, when Y represents a sulphur atom, $R^7$ preferably represents a lower alkyl group, especially a methyl group, and when Y represents an oxygen atom, $R^7$ preferably represents a methyl or phenyl group, especially a substituted phenyl group, for example, a mono-substituted phenyl group, especially a tolyl group or a nitro-phenyl group, for example, an ortho-nitrophenyl group.

The reaction between compound VII and compound IIa, IIIa, IVa, VIa or VIII and also between a resulting compound IVb or VIb and compound Xa or Xb respectively, is generally carried out in a solvent, for example, an organic solvent, for example, a lower alcohol, for example, methanol, ethanol or isopropanol, dimethylformamide, tetrahydrofuran or acetonitrile, or in a mixture of two or more of such solvents. The reaction is generally carried out at a temperature within the range of from 5 to 75°C.

Cyclisation of compound IX may be carried out using an acidic medium, for example, aqueous hydrochloric acid and an organic solvent, for example, an alcohol or a ketone especially acetone, at a temperature within the range of from 10 to 50°C, especially at room temperature.

A compound of formula VII may be produced from a compound of formula XI, XII or XII

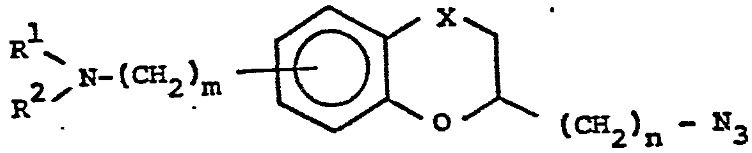

(XI)

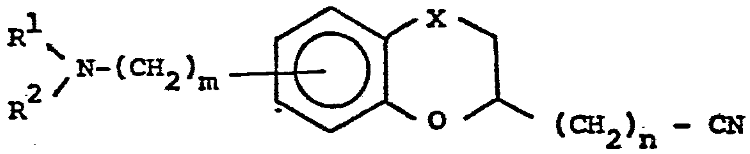

(XII)

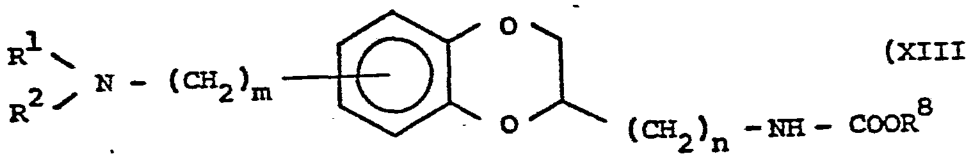

(XIII)

in which formulae $R^1$, $R^2$, Q, X and n are as defined above, and $R^8$ represents a carboxylic acid protecting group, for example, as described in McOmie (loc. cit.) Chapter 5 pages 183-216 and in Greene (loc. cit.) Chapter 5 pages 152-192, which are incorporated herein by reference, for example, lower alkyl, benzyl, p-nitro-benzyl and, especially, t-butyl groups.

Compound XI is converted to compound VII by reduction, for example, using lithium aluminium hydride, sodium borohydride, or hydrogen in the presence of a hydrogenation catalyst, for example, 10% palladium on charcoal.

Compound XII may be converted into compound VII by reduction in the presence of a base, for example, ammonia. Hydrogen and an appropriate hydrogenation catalyst are generally used, for example, rhodium on carbon, for example 5% Rh/C, and the reaction is preferably carried out in a solvent or diluent, for example, an alcohol, for example, methanol or ethanol saturated with ammonia.

Compound XIII may be converted into a compound of formula VII by hydrolysis in the presence of an acid, for example, hydrochloric acid.

Compounds of formula XI may be prepared according to the following Reaction Scheme 1:

Scheme 1



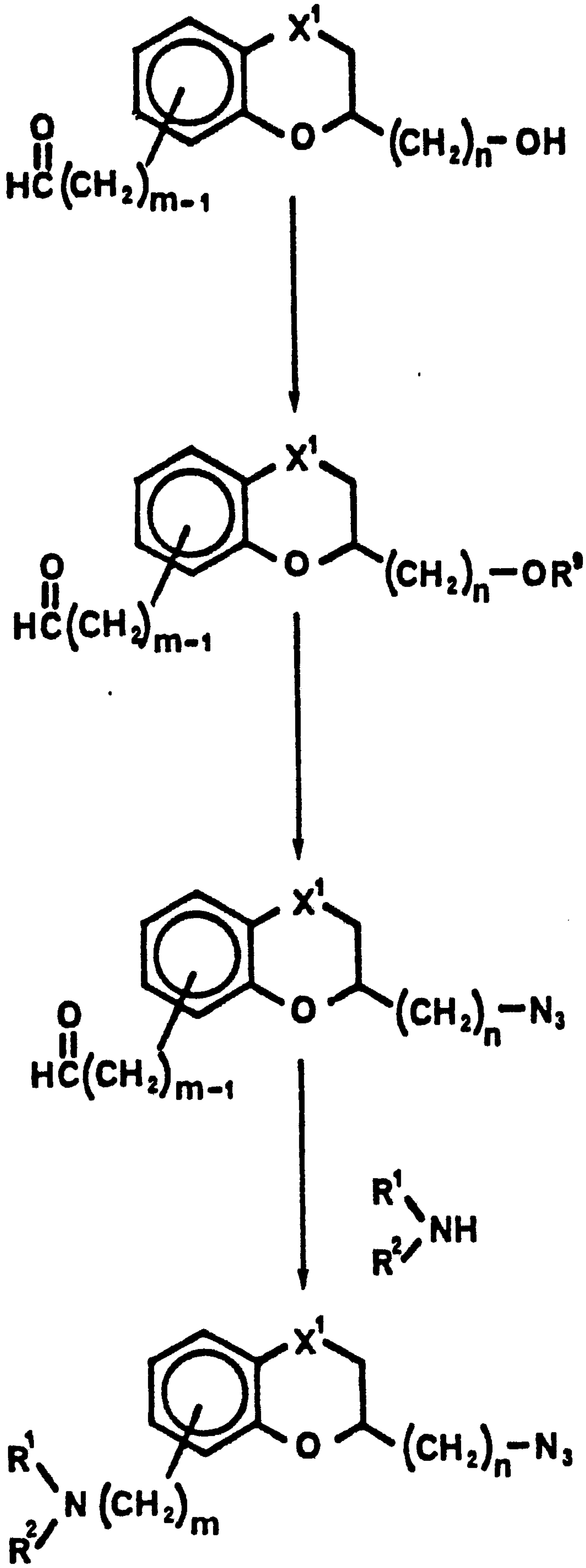

XVII

XVI

XIV

XV

XIa

in which $R^9$ represents an activating group, for example, a sulphonyl group, for example, a toluenesulphonyl, methanesulphonyl or trifluoromethanesulphonyl group, or a trifluoroacetyl group;

$X^1$ has the meanings given above for X with the exception of an -NH- group;

$R^1$, $R^2$, m and n are as defined above.

All four isomers of compound XVII in which (m-1)=0 and $X^1$ represents an oxygen atom are known, as is their conversion into the corresponding isomers of formula XVI, cf A. Funke and A. Paulsen, Gazz. Chim. Ital. 1961, 91, 1268-1281. Other compounds of formulae XVII and XVI may be prepared analogously. (Methods of activating an alcohol, for example, by conversion to a sulphonate ester, for replacement of the hydroxy group are well known.)

Compound XVI is then converted into compound XIV by reaction with an azide, for example, sodium azide. The reaction is generally carried out in a solvent or diluent, for example, dimethylformamide, dimethyl sulphoxide or acetone, generally at a temperature within the range of from 20 to 100°C, especially 80°C.

Compound XIV may be converted into compound XIa by reductive amination. By such a method, compound XIV is reacted with a compound of formula XV

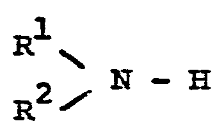

(XV)

in which $R^1$ and $R^2$ are as defined above, in the presence of a reducing agent. The reaction may be carried out with, for example, a borane-pyridine complex or sodium cyanoborohydride, under acidic conditions, and is preferably carried out in a solvent or diluent, for example, an alcohol, for example, methanol. The reaction may be carried out at a temperature within the range of from -10 to 25°C, for example, at room temperature.

To obtain a compound of formula XI wherein X

represents NH, a protecting group $R^4$ may be removed from a resulting compound of formula XIa having a protected NH group. The removal of a nitrogen protecting group $R^4$ is carried out in known manner see, for example, McOmie (<u>loc. cit.</u>) and Greene (<u>loc. cit.</u>). (The term "known" is used herein to mean in actual use in the art or described in the literature of the art.)

Alternatively, compounds of formula XI in which n is the integer 2 and X represents an oxygen atom may be produced according to the following Reaction Scheme 2:

Scheme 2

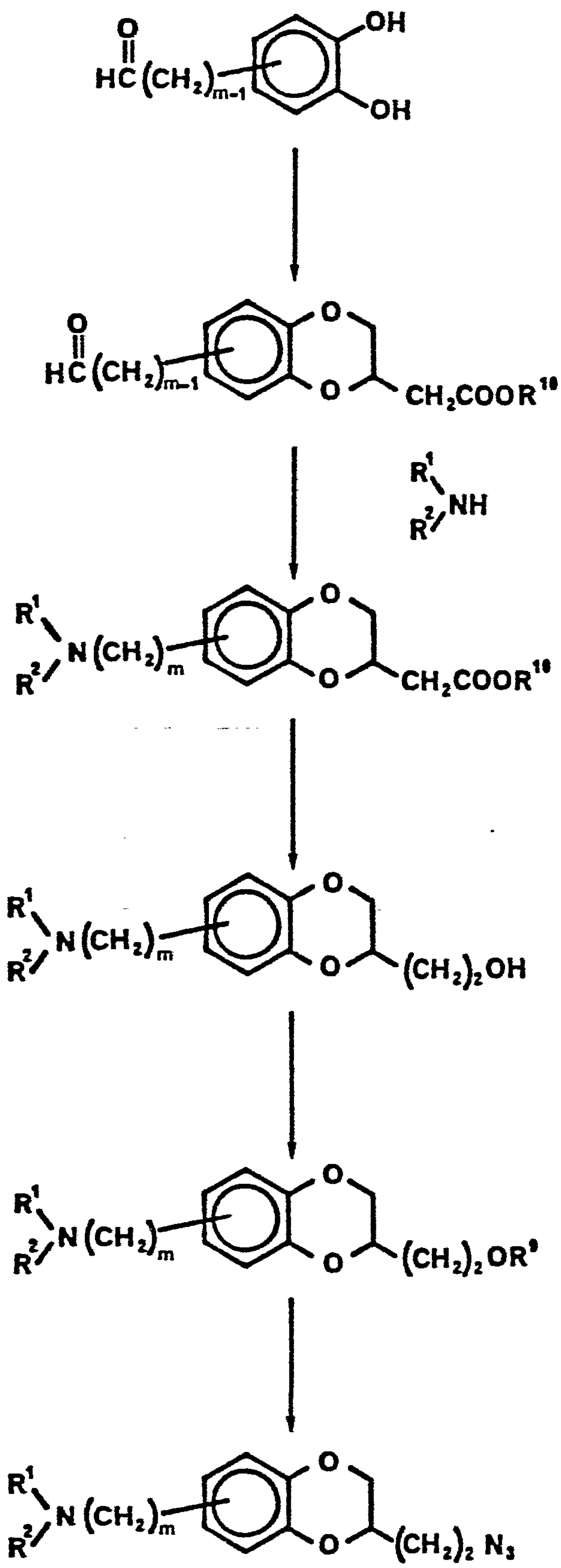

XXII

XXI

XV

XX

XIX

XVIII

XIb

in which $R^1$, $R^2$, $R^9$, m and n are as defined above; and $R^{10}$ represents a carboxylic acid protecting group, as described above in relation to $R^8$.

Compounds of formula XXII are available commercially.

A compound XXII is reacted with a compound of formula XXIII

$$ZCH_2CH{=}CHCOOR^{10} \qquad \text{(XXIII)}$$

in which $R^{10}$ is as defined above and Z represents a halogen atom, especially a bromine atom. The reaction is preferably carried out in the presence of a base, for example, an inorganic base, for example, sodium carbonate. The reaction is generally carried out in a solvent or diluent, for example, dimethylformamide, dimethyl sulphoxide, or a ketone, especially acetone.

The resulting cyclic compound XXI is then reacted with a compound of formula XV as defined in Reaction Scheme 1 to give a compound of formula XX. This reaction is preferably carried out as described in Reaction Scheme I for the reaction of compounds XIV and XV.

The ester of formula XX is then reduced to give compound XIX. The reduction may be carried out using any one of a variety of reducing agents, for example, a metal hydride, for example, lithium aluminium hydride. The reaction is generally carried out in a solvent or diluent, for example, tetrahydrofuran, an ether, or an alcohol, for example, ethanol.

The hydroxy group of compound XIX is then activated, for example, by the introduction of an activating group $R^9$, for example, as described above in Reaction Scheme 1.

The activated compound XIX is then converted into the corresponding azide as described in Reaction Scheme 1.

Compounds of formula XII in which the radical X represents an oxygen atom may be produced according to the following Reaction Scheme 3.

**Scheme 3**

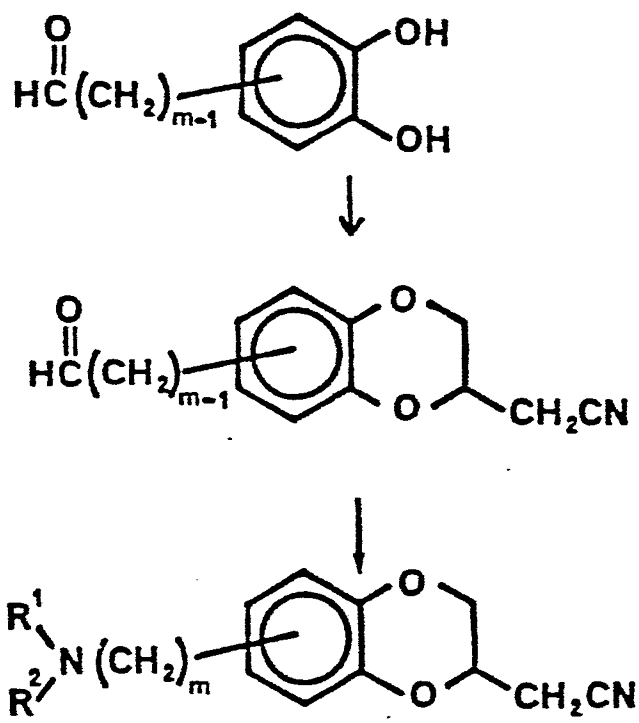

XXV

XXIV

XIIa

in which $R^1$, $R^2$ and m are as defined above.

Compound XXV, which is available commercially, is reacted with a compound XXVI

$$ZCH_2CH{=}CHCN \qquad (XXVI)$$

in which Z is as defined above, to give a compound XXIV. Compound XXVI (in the form of a cis-trans mixture) is described by W.J. Bailey and J. Bello, J. Org. Chem. (1955), 20, 525. The reaction is carried out as decribed above for the reaction of compounds XXII and XXIII. The resulting compound XXIV is then reacted with a compound of formula XV as defined in Reaction Scheme I, under the conditions described in Reaction Scheme I for the reaction of compounds XIV and XV.

Compounds of formula XII in which X represents $NR^4$ may be produced according to the following Reaction Scheme 4:

Scheme 4

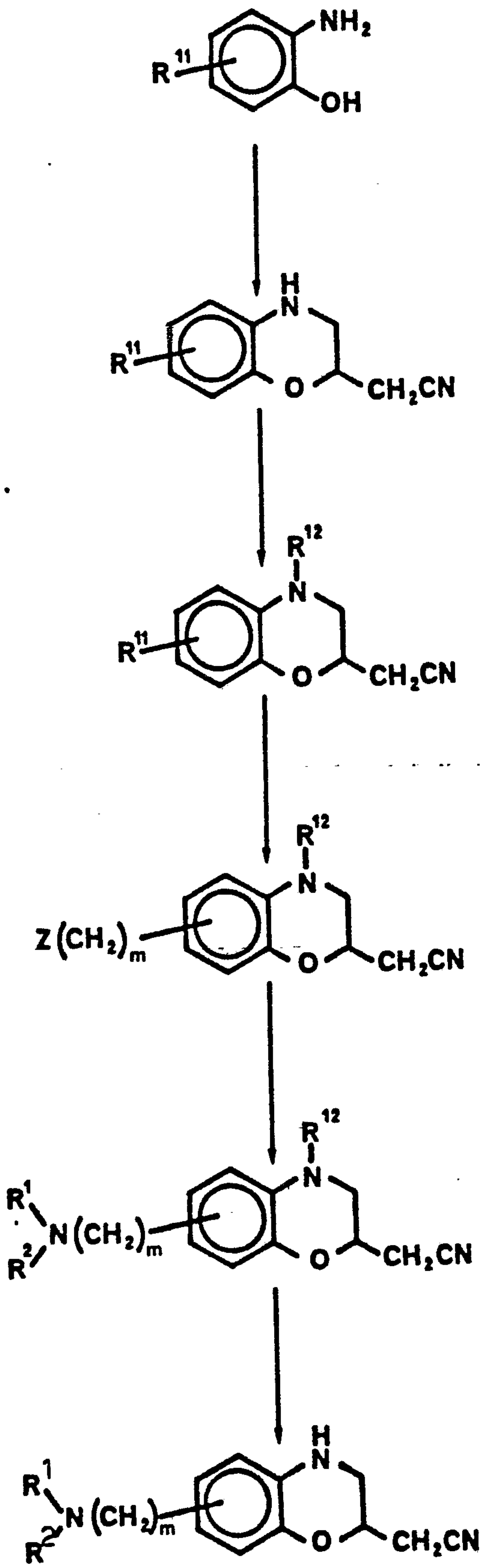

XXXI

XXX

XXIX

XXVIII

XXVII

XIIb

in which $R^1$, $R^2$ and m are as defined above, $R^{11}$ represents an alkyl group, especially a methyl group, and $R^{12}$ represents a nitrogen protecting group, for example, as defined above, for example, a carboxylic acyl group, and especially a formyl group.

A compound of formula XXXI is reacted with a compound of formula XXVI as described in Reaction Scheme 3 to give a compound of formula XXX. This compound is then acylated to protect the nitrogen atom, for example, to introduce a formyl protecting group, formic acid is used. It is preferable to carry out the acylation reaction in the presence of an activating agent, for example, acetic anhydride. The reaction temperature is, for example, in the range of from 20 to 60°C, for example, at room temperature.

The resulting compound XXIX is then halogenated, for example, by means of a reagent capable of free radical halogenation at the group $R^{11}$, for example, there may be used N-bromo-succinimide or a derivative thereof in the presence of light and/or a peroxide, for example, benzoyl peroxide. The halogenation is carried out with heating, and generally in a solvent or diluent, for example, benzene or a chlorinated hydrocarbon, for example, carbon tetrachloride, to give compund XXVIII. This compound may be isolated, if desired, but is generally reacted <u>in situ</u> with compound XV to give compound XXVII.

If $R^{12}$ is a protecting group as defined for $R^4$, then compound XXVII is itself a compound of formula XII. If desired, the protecting group $R^{12}$ may be removed from compound XXVII to give the corresponding -NH- compound of formula XIIb. This reaction is carried out in known manner (see above), for example, by means of a mineral acid, for example, hydrochloric acid. The reaction is generally carried out in a solvent or diluent, for example, an alcohol, for example, methanol.

A compound of formula XIII may be produced according to Reaction Scheme 5 described below:

Scheme 5

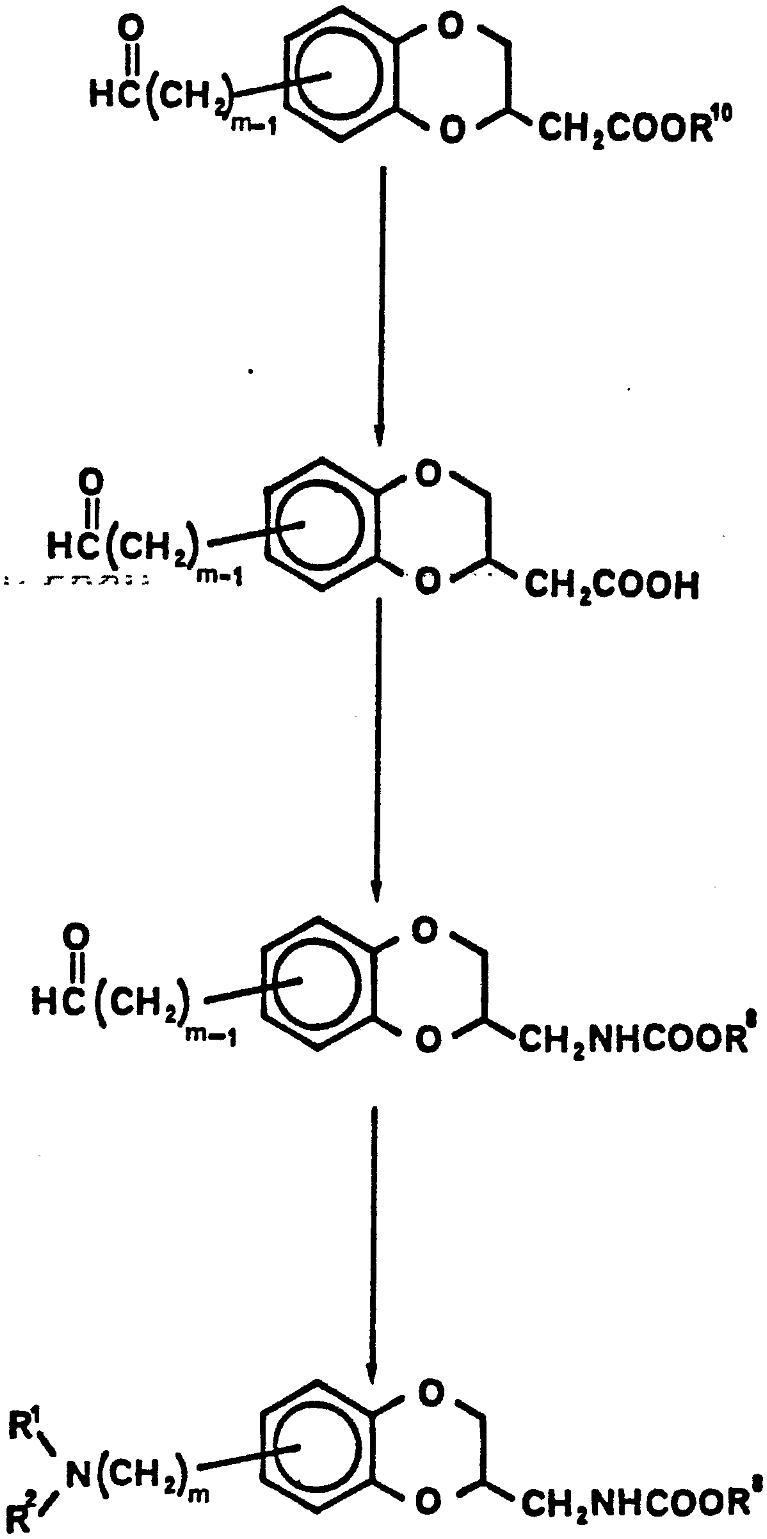

XXI

XXXIII

XXXII

XIII

in which $R^1$, $R^2$, $R^8$ $R^{10}$ and m are as defined above.

Compound XXI, which is described in Reaction Scheme 2, is hydrolysed under acidic or basic conditions, preferably under acidic conditions using, for example, hydrochloric acid, to give compound XXXIII. Compound XXXIII may then be converted into the carbamate XXXII using standard methodology, for example, see C.A. Buehler and D.Pearson, Survey of Organic Synthesis, pages 494-503, Wiley-Interscience, New York,(1970). In the present case it is advantageous to use diphenylphosphorylazide in t-butanol for the conversion (see J. Amer. Chem. Soc. (1972), 94, 6203).

Compound XXXII is then reacted with compound XV as described in Reaction Scheme 1 to give compound XIII. As mentioned above, compound XIII is hydrolysed to give compound VII in which the radical X represents an oxygen atom. The hydrolysis is generally carried out using a mineral acid, for example, hydrochloric acid. The reaction is generally carried out in a solvent or diluent, for example, an alcohol, for example, methanol, at a temperature within the range of from 20 to 100°C, and preferably at reflux temperature.

As indicated above, the compounds of the present invention have histamine H-2 antagonist activity. Accordingly, the present invention provides a pharmaceutical preparation which comprises a compound of the general formula I or a physiologically tolerable salt thereof as active ingredient, in admixture or conjunction with a pharmaceutically suitable carrier. The preparation may be in a form suitable for enteral or parenteral administration, for example, for oral or intravenous administration. The preparation may be in unit dosage form, for example, as tablets or capsules, or in unit or multiple dose ampoules or vials. From 0.1 to 10 mg of the active substance may be administered per kg body weight. A preparation of the invention may also comprise one or more pharmaceutically active substances, for example,

histamine H-1 antagonists.

The present invention also provides the use of a compound of the general formula I for the manufacture of a medicament, especially for use as a histamine H-2 antagonist, and particularly for treating conditions resulting from stimulation by histamine of H-2 receptors, using a compound of formula I alone, for example, in inhibiting gastric acid secretion and thus treating its sequelae, for example, gastric and peptic ulcers, or together with H-1 antagonists, for example, in treating allergic and certain inflammatory conditions.

Examples of preferred compounds of formula I are the following compounds, which are defined in terms of the formula XXXIV, and are also named:

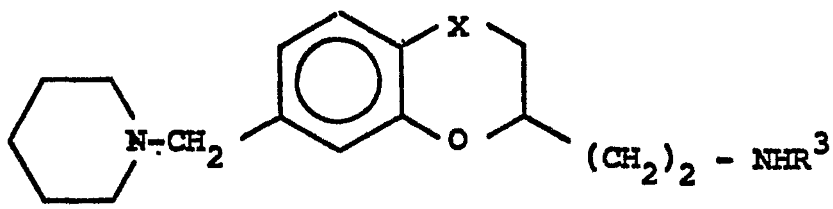

(XXXIV)

3-Amino-1-methyl-5-[2-(7-(N-piperidinylmethyl)-2,3-dihydro[1,4]benzodioxin-2-yl)ethylamino]-1,2,4-triazole ie compound XXXIV in which

X = -O-     $R^3$ =

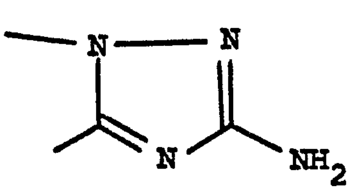

3-Amino-4-methyl-5-[2-(7-(N-piperidinylmethyl)-2,3-dihydro[1,4]benzodioxin-2-yl)ethylamino]-1,2,4,6-thiatriazine 1,1-dioxide ie compound XXXIV in which

X = -O-     $R^3$ =

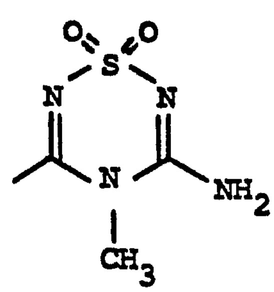

4-Methyl-3-[2-(7-(N-piperidinylmethyl)-2,3-dihydro[1,4]-benzodioxin-2-yl)ethylamino]-5-propargylamino-1,2,4,6-thiatriazine 1,1-dioxide ie compound XXXIV in which

X = -O-  $R^3$ = 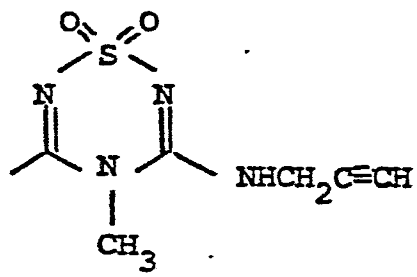

3-Amino-4-methyl-5-[2-(7-(N-piperidinylmethyl)-2,3-dihydro[4H][1,4]benzoxazin-2-yl)ethylamino]-1,2,4,6-thiatriazine 1,1-dioxide ie compound XXXIV in which

X = -NH-  $R^3$ = 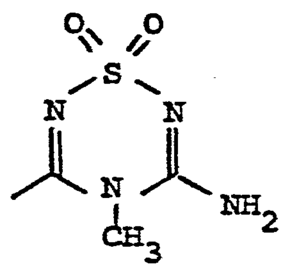

In the above compounds and throughout the present specification, the terms "2,3-dihydro[1,4]benzodioxin and "2,3-dihydro[4H][1,4]benzoxazin" are used to denote the following ring structures A and B respectively.

(A) 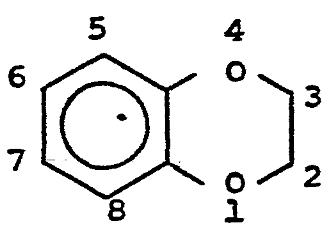

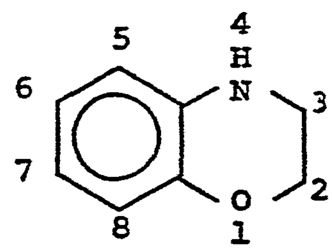 (B)

The compounds of formulae VII, IX, XI, XII, XIII, XIV, XVIII, XIX, XX, XXI, XXII, XXIV, XXVIII, XXIX, XXX, XXXII and XXXIII are part of the present invention. These compounds may exist in various isomeric and/or tautomeric forms, and the present invention includes all such forms.

The following examples illustrate the invention, but are not limiting.

Melting points were determined on a Reichert hot-stage apparatus; temperatures are expressed in degrees Celcius; and unless stated otherwise, NMR spectra (250 MHz) were determined in $CDCl_3$. Thin layer chromatography (TLC) was carried out on silica using the solvents given in the text.

EXAMPLE 1

2,3-Dihydro-2-(toluenesulphonyloxymethyl)[1,4]benzodioxin-8-carboxaldehyde

p-Toluenesulphonyl chloride (4.8g) was added portionwise to a solution of 2,3-dihydro-2-(hydroxymethyl)[1,4]benzodioxin-8-carboxaldehyde (5.0g) in pyridine (20ml) cooled in ice. When the addition was complete (5 minutes) the mixture was warmed to room temperature, stirred for 3 hours and then partitioned between 1N aqueous hydrochloric acid and ethyl acetate. The organic layer was separated, washed with 1N HCl, dried over anhydrous magnesium sulphate and evaporated to give a colourless solid which was recrystallised from isopropanol to give 4.9g of the title compound.

$\nu_{max}$ (Nujol*) 1675, 1600 $cm^{-1}$

$\delta$ 2.45 (3H, s); 4.18-4.25 (1H, m); 4.35-4.45 (1H, m); 4.38 (2H, s); 4.55-4.65 (1H, m); 6.95-7.90 (7H, m); 10.15 (1H, s).

*"Nujol" is a Trade Mark

EXAMPLE 2

2-(Azidomethyl)-2,3-dihydro[1,4]benzodioxin-8-carboxaldehyde

A suspension of sodium azide (1.3g) in a solution of 2,3-dihydro-2-(p-toluenesulphonyloxymethyl)[1,4]benzodioxin-8-carboxaldehyde (4.0g) in dimethylformamide (20ml) was stirred with heating at 80°C for 16 hours. The mixture was then evaporated to dryness, and the residue

partitioned between ethyl acetate and water. The organic phase was separated, washed with 1N sodium hydroxide solution, dried ($MgSO_4$) and evaporated to give a colourless solid which was recrystallised from isopropanol to give 1.9g of the title compound.

$\nu_{max}$ (Nujol) 2080, 1675, 1600, 1590 $cm^{-1}$

$\delta$ 3.6-3.75 (2H, m); 4.11-4.18 (1H, m); 4.30-4.36 (1H, m); 4.50-4.60 (1H, m); 6.95 (1H, t, J 9Hz); 7.16 (1H, d, J 9Hz); 7.46 (1H, d, J 9Hz); 10.41 (1H, s).

EXAMPLE 3

2-(Azidomethyl)-2,3-dihydro[1,4]benzodioxin-6-carboxaldehyde

The title compound was obtained as an oil from 2,3-dihydro-2-(p-toluenesulphonyloxymethyl)[1,4]benzodioxin-6-carboxaldehyde by a procedure analogous to that described in Example 2.

$\nu_{max}$ (film) 2100, 1685, 1600, 1585 $cm^{-1}$

$\delta$ 3.55-3.65 (2H, m); 4.06-4.16 (1H, m); 4.30-4.35 (1H, m); 4.35-4.50 (1H, m); 7.05 and 7.45 (2H, ABq, J 9Hz); 7.42 (1H, s); 9.89 (1H, s).

EXAMPLE 4

2-Azidomethyl-2,3-dihydro-8-(N-piperidinylmethyl)-[1,4]benzodioxin

Piperidine (3.5g) was added in one portion to a solution of 2-azidomethyl-2,3-dihydro[1,4]benzodioxin-8-carboxaldehyde (1.5g) in methanol (30ml) cooled in ice. A solution of hydrogen chloride in methanol (3.44M, 4.1 ml) was then added, followed by sodium cyanoborohydride (0.88g), and the resulting mixture was stirred for 18 hours at room temperature. The solvent was evaporated and the residue treated with 2N HCl (30ml). The solution was extracted with ethyl acetate and the pH adjusted to 14 with sodium hydroxide pellets. The resulting solution was extracted with ethyl acetate, the organic layer separated, dried ($MgSO_4$) and evaporated to afford the title compound

as a yellow oil. Pure material (1.74g) was obtained by column chromatography on silica using chloroform/methanol (95:5) as solvent.

$\delta$ 1.35-1.50 (2H, m); 1.50-1.65 (4H, m); 2.42-2.52 (4H, m); 3.45-3.64 (2H, m); 4.02-4.12 (1H, m); 4.22-4.30 (1H, m); 4.30-4.45 (1H, m); 6.80-7.05 (3H, m).

EXAMPLE 5

2-Azidomethyl-2,3-dihydro-6-(N-piperidinylmethyl)-[1,4]benzodioxin

The title compound was obtained as a colourless oil from 2-azidomethyl-2,3-dihydro-[1,4]benzodioxin-6-carboxaldehyde by a procedure analogous to that described in Example 4.

$\nu_{max}$ (film) 2100, 1590 $cm^{-1}$

EXAMPLE 6

2,3-Dihydro-5-formyl[1,4]benzodioxin-2-acetic acid methyl ester

A heterogeneous mixture of 2,3-dihydroxybenzaldehyde (6.9g), methyl 4-bromocrotonate (9.8g) and potassium carbonate (15g) in dimethyl formamide (100ml) was stirred at room temperature for 18 hours. The solvent was subsequently evaporated and the residue partitioned between diethyl ether and 1N sodium hydroxide solution. The organic phase was separated, washed with 1N sodium hydroxide solution, then with water, was dried over anhydrous magnesium sulphate and evaporated to give a yellow oil. Bulb to bulb distillation afforded 6.6g of the title compound, b.p. 150-170°C. 0.5 mm Hg.

$\delta$ 2.66-2.94 (2H, m); 3.77 (3H, s); 4.10-4.20 (1H, m); 4.48-4.56 (1H, m); 4.70-4.80 (1H, m); 7.0 (1H, t, J 9Hz); 7.17 (1H, d, J 9Hz); 7.46 (1H, d, J 9Hz); 10.36 (1H, s).

EXAMPLE 7

2,3-Dihydro-7-formyl[1,4]benzodioxin-2-acetic acid methyl ester

The title compound was obtained from 3,4-dihydroxybenzaldehyde and methyl 4-bromocrotonate by a procedure analogous to that described in Example 6.

δ 2.64-2.92 (2H, m); 3.82 (3H, m); 4.08-4.16 (1H, m); 4.44-4.50 (1H, m); 4.64-4.75 (1H, m); 7.06 (1H, d, J 9Hz); 7.48 (1H, s); 7.50 (1H, d, J 9Hz); 9.90 (1H, s).

EXAMPLE 8

2,3-Dihydro-5-(N-piperidinylmethyl)[1,4]benzodioxin-2-acetic acid methyl ester

The title compound was obtained from 2,3-dihydro-5-formyl[1,4]benzodioxin-2-acetic acid methyl ester by a procedure analogous to that described in Example 4.

δ 1.38-1.50 (2H, m); 1.54-1.65 (4H, m) 2.40-2.50 (4H, m); 2.60-2.86 (2H, m); 3.52 and 3.62 (2H, ABq, J 12Hz); 3.78 (3H, s); 3.96-4.04 (1H, m); 4.35-4.41 (1H, m); 4.60-4.71 (1H, m); 6.78-7.02 (3H, m).

EXAMPLE 9

2,3-Dihydro-7-(N-piperidinylmethyl)[1,4]benzodioxin-2-acetic acid methyl ester

The title compound was obtained as a yellow oil from 2,3-dihydro-7-formyl[1,4]benzodioxin-2-acetic acid methyl ester by a procedure analogous to that described in Example 4.

δ 1.35-1.50 (2H, m); 1.50-1.65 (4H, m); 2.30-2.40 (4H, m); 2.58-2.85 (2H, m); 3.38 (2H, s); 3.76 (3H, s); 3.94-4.04 (1H, m); 4.28-4.35 (1H, m); 4.56-4.70 (1H, m); 6.80-6.90 (3H, m).

EXAMPLE 10

2,3-Dihydro-2-(2-methanesulphonyloxyethyl)-5-(N-piperidinylmethyl)[1,4]benzodioxin

A solution of 2,3-dihydro-5-(N-piperidinylmethyl)-[1,4]benzodioxin-2-acetic acid methyl ester (6.6g) in dry ether (50ml) was added dropwise to a suspension of lithuim aluminium hydride (3.8g) in ether (250ml) cooled in ice such that the temperature of the reaction did not exceed 25°C. When the addition was complete (30 minutes), the ether was heated to reflux temperature and maintained at that temperature for 90 minutes. The mixture was then cooled in ice and the excess lithium aluminium hydride destroyed by the addition of ethyl acetate. Water (25ml) was then added and the resulting white precipitate removd by filtration through Celite (Trade Mark). The organic phase was separated, washed with brine, dried ($MgSO_4$) and evaporated to give 6.0g of 2,3-dihydro-2-(2-hydroxyethyl)-5-(N-piperidinylmethyl)[1,4]benzodioxin as a pale yellow oil, which appeared as a single spot on TLC using chloroform/methanol (9:1) as solvent.

The crude product (5.2g) was taken up in methylene chloride (150ml). Triethylamine (3.5g) and methanesulphonyl chloride (3.4g) were added successively, and the resulting mixture was stirred at room temperature for 18 hours. The mixture was then washed with a 2N sodium carbonate solution (20ml), water, (20ml), and dried ($MgSO_4$). Evaporation afforded 4.5g of material comprising a mixture of the title compound and the starting material. The desired compound was purified by column chromatograpy on silica using chloroform/methanol mixtures as eluant to afford 3.35g of the title compound.

$\delta$ 1.38-1.50 (2H, m); 1.50-1.64 (4H, m); 2.02-2.14 (2H, m); 2.40-2.50 (4H, m); 3.08 (3H, s); 3.46-3.62 (2H, m); 3.90-4.00 (1H, m); 4.28-4.40 (2H, m); 4.46-4.55 (2H, m); 6.78-7.00 (3H, m).

EXAMPLE 11

2,3-Dihydro-2-(2-methanesulphonyloxyethyl)-7-(N-piperidinylmethyl)[1,4]benzodioxin

The title compound was obtained from 2,3-dihydro-7-(N-piperidinylmethyl)[1,4]benzodioxin-2-acetic acid methyl ester by a procedure analogous to that described in Example 10.

δ 1.38-1.50 (2H, m); 1.50-1.64 (4H, m); 2.04-2.14 (2H, m); 2.35-2.42 (4H, m); 3.08 (3H, s); 3.40 (2H, s); 3.92-4.00 (1H, m); 4.25-4.30 (1H, m); 4.30-4.42 (1H, m); 4.46-4.54 (2H, m); 6.82-6.92 (3H, m).

EXAMPLE 12

2-(2-Azidoethyl)-2,3-dihydro-5-(N-piperidinylmethyl)[1,4]-benzodioxin

The title compound was obtained as an oil from 2,3-dihydro-2-(2-methanesulphonyloxyethyl)-5-(N-piperidinylmethyl)[1,4]benzodioxin by a procedure analogous to that described in Example 2.

$\nu_{max}$ (film) 2100, 1600 $cm^{-1}$

δ 1.35-1.48 (2H, m); 1.52-1.64 (4H, m); 1.75-2.00 (2H, m); 2.36-2.50 (4H, m); 2.40-2.65 (4H, m); 3.88-3.96 (1H, m); 4.25-4.35 (2H, m); 6.78-6.98 (3H, m).

EXAMPLE 13

2-(2-Azidoethyl)-2,3-dihydro-7-(N-piperidinylmethyl)[1,4]-benzodioxin

The title compound was obtained as an oil from 2,3-dihydro-2-(2-methanesulphonyloxyethyl)-7-(N-piperidinylmethyl)[1,4]benzodioxin by a procedure analogous to that described in Example 2.

$\nu_{max}$ (film) 2100, 1580 $cm^{-1}$

δ 1.40-1.50 (2H, m); 1.52-1.65 (4H, m); 1.82-2.02 (2H, m); 2.35-2.45 (4H, m); 3.41 (2H, s); 3.58-3.65 (2H, m); 3.90-4.00 (1H, m); 4.25-4.35 (2H, m); 6.80-6.92 (3H, m).

EXAMPLE 14

2,3-Dihydro-5-formyl[1,4]benzodioxin-2-acetonitrile

The title compound was obtained as an oil from 2,3-dihydroxybenzaldehyde and 4-bromocrotonitrile by a procedure analogous to that described in Example 6.

δ 2.86 (2H, d, J 6Hz); 4.25-4.35 (1H, m); 4.50-4.70

(2H, m); 6.98 (1H, t, J 7Hz); 7.22 (1H, d, J 7Hz); 7.52 (1H, d, J 7Hz); 10.35 (1H, s).

EXAMPLE 15

2,3-Dihydro-7-formyl[1,4]benzodioxin-2-acetonitrile

The title compound was obtained as colourless crystals (m.p. 93-95°C.) from 3,4-dihydroxybenzaldehyde and 4-bromocrotonitrile by a procedure analogous to that described in Example 6.

$\nu_{max}$ (film) 2240, 1695, 1275 $cm^{-1}$

$\delta$ 2.84 (2H, d, J 6Hz); 4.14-4.21 (1H, m); 4.41-4.46 (1H, m); 4.52-4.64 (1H, m); 7.05 (1H, d, J 9Hz); 7.50-7.55 (2H, m); 9.84 (1H, s).

EXAMPLE 16

2,3-Dihydro-5-(N-piperidinylmethyl)[1,4]benzodioxin-2-acetonitrile

The title compound was obtained as an oil from 2,3-dihydro-5-formyl[1,4]benzodioxin-2-acetonitrile by a procedure analogous to that described in Example 4. The product was shown to be homogeneous by TLC using chloroform/methanol/ammonia (9:1:0.1) as solvent.

$\delta$ 1.38-1.50 (2H, m); 1.54-1.65 (4H, m); 2.40-2.50 (4H, m); 2.82 (2H, d, J 6Hz); 3.48-3.64 (2H, m); 4.08-4.16 (1H, m); 4.35-4.42 (1H, m); 4.50-4.60 (1H, m); 6.84-7.08 (3H, m).

EXAMPLE 17

2,3-Dihydro-7-(N-piperidinylmethyl)[1,4]benzodioxin-2-acetonitrile

The title compound was obtained as an oil from 2,3-dihydro-7-formyl[1,4]benzodioxin-2-acetonitrile by a procedure analogous to that described in Example 4. The product was shown to be homogeneous by TLC using chloroform/methanol (9:1) as solvent.

$\delta$ 1.38-1.50 (2H, m); 1.50-1.65 (4H, m); 2.55-2.64 (4H, m); 2.82 (2H, d, J 6Hz); 3.38 (2H, s); 4.05-4.60 (3H, m); 6.85-6.94 (3H, m).

EXAMPLE 18

2,3-Dihydro-7-methyl-[4H][1,4]benzoxazine-2-acetonitrile

A mixture of 6-amino-3-methylphenol (12g), 4-bromocrotonitrile (15.32g) and sodium hydrogen carbonate (7.36g) in dry methanol (300ml) was stirred at ambient temperature for 16 hours. Potassium carbonate (120mg) was added and stirring continued at ambient tempeature for 45 minutes. The mixture was filtered and the solvent evaporated to give an oil which was partitioned between ether and 1N NaOH solution. The organic phase was separated and extracted with 1N HCl. The acidic phase was neutralised and the product extracted with ether. The ether extract was dried ($MgSO_4$)and evaporated to give 7.6g of the title compound as a red oil.

$\nu_{max}$ (film) 3400, 2250, 1620, 1590 $cm^{-1}$

$\delta$ 2.22 (3H, s); 2.76 (2H, d, J 6Hz); 3.24-3.34 (1H, m); 3.45-3.54 (1H, m); 3.60-3.80 (1H, broad s); 4.44-4.54 H, m); 6.53 and 6.63 (2H, ABq, J 9Hz); 6.65 (1H, s). m/e 188 ($m^+$)

EXAMPLE 19

2,3-Dihydro—4-formyl-7-methyl[1,4]benzoxazine-2-acetonitrile

Acetic anhydride (11.1ml) was added dropwise to a solution of 2,3-dihydro-7-methyl-[4H][1,4]benzoxazine-2-acetonitrile (2.5g) in 98% formic acid (34ml) such that the temperature did not exceed 60°C. The resulting mixture was stirred at ambient temperature for 3 hours and then quenched with ice-water (12ml). The mixture was reduced in volume and partitioned between chloroform and 1N NaOH solution. The organic phase was separated, washed with 1N NaOH, with brine, and with water, dried ($MgSO_4$), and evaporated to give 3g of a red oil which partially solidified on standing.

$\nu_{max}$ (film) 3500, 3320, 2250, 1670 $cm^{-1}$

$\delta$ 2.32 (3H,s); 2.75-2.97 (2H, m); 3.38-3.46 (1H, m); 4.36-4.55 (2H, m); 6.84 and 7.15 (2H, ABq, J 9Hz); 6.89 (1H, s). m/e 216 ($m^+$)

EXAMPLE 20

2,3-Dihydro-4-formyl-7-(N-piperidinylmethyl)[1,4]-benzoxazine-2-acetonitrile

A solution of 2,3-dihydro-4-formyl-7-methyl[1,4]-benzoxazine-2-acetonitrile (1g) in carbon tetrachloride (40ml) was heated at reflux and a mixture of N-bromosuccinimde (0.83g) and benzoyl peroxide (5 mg) was added portionwise over 10 minutes. The resulting solution was heated for 3 hours, cooled to room temperature, and chloroform was added until the mixture was homogeneous. The mixture was washed with sodium carbonate solution (twice), and with brine, dried ($MgSO_4$), and evaporated to afford a yellow oil. The oil was taken up in dry methanol (50ml), piperidine (0.91ml) was added and the mixture stirred at ambient temperature for 1 hour. The solvent was evaporated and the residual oil subjected to column chromatography on silica using chloroform/methanol mixtures as eluant to give 0.26g of the title compound as a pale yellow solid, m.p. 114-116°C.

$\nu_{max}$ ($CHCl_3$ solution) 2900-2600, 2230, 1675 $cm^{-1}$

$\delta$ 1.36-1.50 (2H, m); 1.50-1.65 (4H, m); 2.30-2.42 (4H, m); 2.70-2.90 (2H, m); 3.30-3.40 (1H, m); 3.40 (2H, s); 4.30-4.54 (2H, m); 6.94 and 7.13 (2H, ABq, J 9Hz), 7.00 (1H, s); 8.83 (1H, s).

m/e 299 ($m^+$)

EXAMPLE 21

2,3-Dihydro-7-(N-piperidinylmethyl)-[4H][1,4]-benzoxazine-2-acetonitrile.Hydrochloride salt

2,3-Dihydro-4-formyl-7-(N-piperidinylmethyl)[1,4]-benzoxazine-2-acetonitrile (50mg) was stirred in methanol (5ml) containing 1N HCl (0.2ml) at ambient temperature for 4 days. The solvent was evaporated and the residue subjected to column chromatography on silica using chloroform/methanol mixtures as eluant, to afford 20 mg of the title compound as an oily product.

$\nu_{max}$ ($CHCl_3$) 3400, 3000-2300, 2230, 1620, 1590 $cm^{-1}$

$\delta$ 1.45-1.56 (2H, m); 1.73-1.83 (4H, m); 2.60-2.72 (4H,

m); 2.80 (2H, d, J 6Hz); 3.25-3.58 (2H, m); 3.63 (2H, s); 4.43-4.53 (1H, m); 6.63 and 6.91 (2H, ABq, J 9Hz); 7.84 (1H, s).

m/e 271 ($m^+$)

EXAMPLE 22

2,3-Dihydro-7-formyl[1,4]benzodioxin-2-acetic acid

2,3-Dihydro-7-formyl[1,4]benzodioxin-2-acetic acid methyl ester (5.9g) was heated at reflux in 10% HCl solution (40ml) for 2 hours. The reaction mixture was subsequently basified, extracted with chloroform and the aqueous phase reacidified and extracted with ether. The organic phase was separated, washed with brine, dried ($MgSO_4$) and evaporated to afford 3.08g of the title compound as a yellow solid, m.p. 158-161°C.

δ 2.71-2.96 (2H, m); 4.07-4.14 (1H, m); 4.38-4.47 (1H, m); 4.65-4.75 (1H, m); 7.04 (1H, d, J 9Hz); 7.45-7.51 (2H, m); 9.85 (1H, s).

EXAMPLE 23

2-Aminomethyl-2,3-dihydro-7-formyl[1,4]benzodioxin t-butyl carbamate

A mixture of 2,3-dihydro-7-formyl[1,4]benzodioxin-2-acetic acid (0.5g), triethylamine (0.32ml) and diphenylphosphoryl azide (0.48ml) in dry t-butanol was heated at reflux for 24 hours. The volatile material was removed by evaporation and the residue taken up in ethyl acetate. The ethyl acetate solution was washed successively with 5% citric acid solution, water, saturated sodium bicarbonate solution, and brine, dried ($MgSO_4$) and evaporated to give a yellow oil. Column chromatography on silica using chloroform as eluant afforded 250 mg of the title compound.

δ 1.49 (9H, s); 3.40-3.60 (2H, m); 4.02-4.12 (1H, m); 4.25-4.36 (1H, m); 4.38-4.46 (1H, m); 4.95-5.10 (1H, broad s); 7.05 (1H, d, J 9Hz); 7.45-7.50 (2H, m); 9.90 (1H, s).

EXAMPLE 24

2-Aminomethyl-2,3-dihydro-7-(N-piperidinylmethyl)[1,4]-benzodioxin t-butyl carbamate

2-Aminomethyl-2,3-dihydro-7-formyl[1,4]benzodioxin t-butyl carbamate (081g) was dissolved in methylene chloride (10ml) to which was added sequentially piperidine (1.64ml) and glacial acetic acid (2ml). Borane-pyridine complex (0.3ml) was added and the mixture was stirred at ambient temperature for 18 hours. The solution was basified with 2.5M NaOH solution and extracted twice with ethyl acetate. The combined organic extracts were washed with brine, dried ($MgSO_4$), and evaporated to give a yellow oil. Column chromatography on silica with chloroform/methanol mixtures as eluant afforded 0.78g of the title compound as a yellow oil, which showed as a single spot on TLC using chloroform/methanol (3.1) as solvent.
$\delta$ 1.30-1.50 (6H, m); 1.36 (9H, s); 2.30-2.40 (4H, m); 3.27 (2H, s); 3.30-3.40 (2H, m); 3.80-3.90 (1H, m); 4.10-4.22 (2H, m); 5.17 (1H, broad s); 6.70-6.80 (3H, m).

EXAMPLE 25

2-(2-Aminoethyl)-2,3-dihydro-5-(N-piperidinylmethyl)[1,4]-benzodioxin

Method A

2-(2-Azidoethyl)-2,3-dihydro-5-(N-piperidinylmethyl)-[1,4]benzodioxin (2.1g) im methanol (50ml) was hydrogenated over 10% palladium/charcoal (0.5g) at about 200 kPa (30 psi) for 2 hours. The catalyst was removed and the solvent evaporated to afford 1.8g of the title compound as a colourless oil.

Method B

2,3-Dihydro-5-(N-piperidinylmethyl)[1,4]benzodioxin-2-acetonitrile (0.38g)was taken up in ammoniacal methanol (50ml) and hydrogenated over 5% rhodium/carbon (0.3g) at about 235 kPa (35psi) for 3 hours. Chloroform (50ml) was

added, the catalyst removed by filtration and the filtrate evaporated to afford 0.25g of the title compound as a colourless oil identical with the material obtained by Method A.

$\nu_{max}$ 3500-3100 (broad), 1570, 750 $cm^{-1}$

$\delta$ 1.40-1.54 (2H, m); 1.65-1.78 (4H, m); 1.78-1.92 (2H, m); 2.55-2.65 (4H, m); 3.00-3.20 (4H, m); 3.64-3.80 (2H, m); 3.90-4.00 (1H, m); 4.25-4.40 (2H, m); 6.85-7.05 (3H, m).

EXAMPLE 26

2-(2-Aminoethyl)-2,3-dihydro-7-(N-piperidinylmethyl)[1,4]-benzodioxin

The title compound was obtained from 2-(2-azidoethyl)-2,3-dihydro-7-(N-piperidinylmethyl)[1,4]benzodioxin by a procedure analogous to that described in Example 25, Method A, and from 2,3-dihydro-7-(N-piperidinylmethyl)-[1,4]benzodioxin-2-acetonitrile by a procedure analogous to that described in Example 25, Method B.

$\delta$ 1.36-1.48 (2H, m); 1.48-1.55 (4H, m); 1.55-1.90 (4H, m); 2.35-2.46 (2H, m); 2.95-3.05 (2H, m); 3.44 (2H, s); 3.90-4.00 (1H, m); 4.22-4.52 (2H, m); 6.80-6.95 (3H, m).

EXAMPLE 27

2-(2-Aminomethyl)-2,3-dihydro-8-(N-piperidinylmethyl)-[1,4]benzodioxin

The title compound was obtained as a colourless oil from 2-(2-azidomethyl)-2,3-dihydro-8-(N-piperidinylmethyl)[1,4]benzodioxin by a procedure analogous to that described in Example 25, Method A.

(DMSO-$d_6$) $\delta$ 1.38-1.50 (2H, m); 1.50-1.65 (4H, m); 2.55-2.65 (4H, m); 2.90-3.25 (2H, m); 3.50-3.85 (2H, m); 4.08-4.15 (1H, m); 4.36-4.45 (1H, m); 4.50-4.60 (1H, m); 6.85-7.00 (3H, m).

EXAMPLE 28

2-(2-Aminomethyl)-2,3-dihydro-6-(N-piperidinylmethyl)-[1,4]benzodioxin

The title compound was obtained from 2-(2-azidomethyl)-2,3-dihydro-6-(N-piperidinylmethyl)[1,4]benzodioxin by a procedure analogous to that described in Example 25, Method A. The product was a colourless oil b.p. 170-180 °C, 0.8 mmHg

EXAMPLE 29

2-(2-Aminomethyl)-2,3-dihydro-7-(N-piperidinylmethyl)-[1,4]benzodioxin

A solution of 2-aminomethyl-2,3-dihydro-7-(N-piperidinylmethyl)[1,4]benzodioxin *t*-butyl carbamate (0.77g) in 5M HCl (5ml) was stirred at ambient temperature for 18 hours. The reaction mixture was basified with potassium hydroxide pellets and extracted with ethyl acetate. The organic phase was separated and dried ($MgSO_4$) and evaporated to give 0.37g of a colourless oil, which appeared as a single spot on TLC using chloroform/methanol (3:1) as solvent.

$\delta$ 1.35-1.42 (2H, m); 1.42-1.54 (4H, m); 1.60-1.70 (2H, broad s); 2.30-2.40 (4H, m); 2.97 (2H, d, J 6Hz); 3.37 (2H, s); 3.94-4.30 (3H, m); 6.75-6.88 (3H, m).

EXAMPLE 30

2-(2-Aminoethyl)-7-(N-piperidinylmethyl)-2,3-dihydro-[4H][1,4]benzoxazine.Hydrochloride salt

2,3-Dihydro-7-(N-piperidinylmethyl)[4H][1,4]benzoxazin-2-acetonitrile hydrochloride (200 mg) was taken up in methanol (30ml) and hydrogenated over 5% rhodium/carbon (400mg) on a Parr (Trade Mark) apparatus at about 345 kPa (50psi) for 18 hours. The reaction mixture was filtered and the solvent evaporated to afford the title compound as a yellow oil (116mg).

$\delta$ ($CD_3OD$) 1.60-1.70 (2H, m); 1.75-1.95 (4H, m); 1.95-2.08 (2H, m); 3.05-3.50 (8H, m); 4.10(2H, s); 4.15-4.25 (1H, m); 6.66 and 6.86 (2H, ABq, J 9Hz); 6.96 (1H, s).

EXAMPLE 31

3-[(6-(N-Piperidinylmethyl)-2,3-dihydro[1,4]benzodioxin-2-yl)methylamino]-4-methyl-5-[4-methylphenoxy]-1,2,4,6-thiatriazine 1,1-dioxide

To a stirred solution of 4-methyl-3,5-bis(tolyloxy)-1,2,4,6-thiatriazine 1,1-dioxide (1.4g ) in a 1:1 mixture of acetonitrile and chloroform (50ml) was added 2-aminomethyl-2,3-dihydro-6-(N-piperidinylmethyl)-2,3-dihydro-[1,4]benzodioxin (1.14g). The resulting homogeneous solution was stirred at ambient temperature for 96 hours, evaporated to dryness and the residue chromatographed on silica. Elution with chloroform/methanol/ammonia mixtures gave the title compound as a gum (1.6g). (DMSO-$d_6$) $\delta$ 1.30-1.40 (2H, m); 1.40-1.54 (4H, m); 2.25-2.35 (2H, m); 2.32 (3H, s); 3.34 (2H, d, J 6Hz); 3.48 (3H, s); 3.48-3.60 (2H, m); 4.00-4.10 (1H, m); 4.36-4.44 (2H, m); 6.76-6.88 (3H, m); 7.16 and 7.31 (4H, ABq, J 9Hz), 8.25 (1H, broad s).

EXAMPLE 32

3-[(8-(N-Piperidinylmethyl)-2,3-dihydro[1,4]benzodioxin-2-yl)methylamino]-4-methyl-5-[4-methylphenoxy]-1,2,4,6-thiatriazine 1,1-dioxide

The title compound was obtained by a procedure analogous to that described in Example 31 from the corresponding 8-(N-piperidinylmethyl) benzodioxin isomer. (DMSO-$d_6$) $\delta$ 1.40-1.60 (2H, m); 1.60-1.80 (4H, m); 2.36 (3H, s); 2.70-3.00 (4H, m); 3.56 (3H, s); 3.60-3.68 (2H, m); 4.00-4.60 (3H, m); 6.90-7.05 (3H, m); 7.16 and 7.33 (4H, ABq, J 9Hz); 8.50 (1H, broad s).

EXAMPLE 33

3-[(7-(N-Piperidinylmethyl)-2,3-dihydro[1,4]benzodioxin-2-yl)methylamino]-4-methyl-5-[4-methylphenoxy]-1,2,4,6-thiatriazine 1,1-dioxide

The title compound was obtained by a procedure analogous to that described in Example 31 from the corresponding 7-(N-piperidinylmethyl) benzodioxin isomer.

EXAMPLE 34

3-[(7-(N-Piperidinylmethyl)-2,3-dihydro[1,4]benzodioxin-2-yl)-2-(ethylamino)]-4-methyl-5-[4-methylphenoxy]-1,2,4,6-thiatriazine 1,1-dioxide

The title compound was obtained by a procedure analogous to that described in Example 31 from 2-(2-aminoethyl)-2,3-dihydro-7-(N-piperidinylmethyl)-2,3-dihydro-[1,4]benzodioxin.

EXAMPLE 35

3-[(5-(N-Piperidinylmethyl)-2,3-dihydro[1,4]benzodioxin-2-yl)-2-(ethylamino)]-4-methyl-5-[4-methylphenoxy]-1,2,4,6-thiatriazine 1,1-dioxide

The title compound was obtained by a procedure analogous to that described in Example 31 from 2-(2-aminoethyl)-2,3-dihydro-5-(N-piperidinylmethyl)-2,3-dihydro-[1,4]benzodioxin.

EXAMPLE 36

3-[(7-(N-Piperidinylmethyl)[4H]-2,3-dihydro[1,4]benzoxazin-2-yl)-2-(ethylamino)]-4-methyl-5-[4-methylphenoxy]-1,2,4,6-thiatriazine 1,1-dioxide

The title compound was obtained by a procedure analogous to that described in Example 31 from 2-(2-aminoethyl)-2,3-dihydro-7-(N-piperidinylmethyl)[4H]-2,3-dihydro-[1,4]benzoxazine.

($CD_3OD$) $\delta$ 1.50-1.80 (6H, m); 1.90-2.02 (2H, m); 2.34 (3H, s); 2.90-3.00 (4H, m); 3.06-3.16 (1H, m); 3.28-3.40 (1H, m); 3.50 (3H, s); 3.60-4.20 (5H, m); 6.62 and 6.75 (2H, ABq, J 9Hz), 6.82 (1H, s); 7.04-7.30 (4H, m).

EXAMPLE 37

3-[(6-(N-Piperidinylmethyl)-2,3-dihydro[1,4]benzodioxin-2-yl)methylamino]-4-methyl-5-amino-1,2,4,6-thiatriazine 1,1-dioxide

A solution of 3-[(6-(N-piperidinylmethyl)-2,3-dihydro-

benzodioxin-2-yl)methylamino]-4-methyl-5-[4-methylphenoxy]-1,2,4,6-thiatriazine 1,1-dioxide(0.4g) in ammoniacal methanol (160ml) was stirred at ambient temperature for 72 hours. The solvent was evaporated and the residue triturated with ether to affor 0.32g of the title compound as a colourless solid. m.p. 243-245°C. ($DMSO-d_6$) $\delta$ 1.30-1.55 (6H, m); 2.24-2.45 (4H, m); 3.28 (3H, s); 3.98-4.08 (1H, m); 4.35-4.45 (2H, m); 6.76-6.92 (3H, m); 7.44 (2H, broad s); 7.80 (1H, broad t).

EXAMPLE 38

3-[(8-(N-Piperidinylmethyl)-2,3-dihydro[1,4]benzodioxin-2-yl)methylamino]-4-methyl-5-amino-1,2,4,6-thiatriazine 1,1-dioxide

The title compound was obtained as a colourless solid from 3-[(8-(N-piperidinylmethyl)-2,3-dihydro[1,4]-benzodioxin-2-yl)methylamino]-4-methyl-5-[4-methylphenoxy]-1,2,4,6-thiatriazine 1,1-dioxide by a procedure analogous to that described in Example 37. (m.p. 204-206°C.) ($DMSO-d_6$) $\delta$ 1.40-1.70 (6H, m); 2.48-2.58 (4H, m); 3.28 (3H, s); 3.95-4.55 (4H, m); 6.88-7.04 (3H, m); 7.48 (2H, broad s); 8.02 (1H, broad s).

EXAMPLE 39

3-[(7-(N-Piperidinylmethyl)-2,3-dihydro[1,4]benzodioxin-2-yl)methylamino]-4-methyl-5-amino-1,2,4,6-thiatriazine 1,1-dioxide

The title compound was obtained as a colourless solid from 3-[(7-(N-piperidinylmethyl)-2,3-dihydro[1,4]-benzodioxin-2-yl)methylamino]-4-methyl-5-[4-methylphenoxy]-1,2,4,6-thiatriazine 1,1-dioxide by a procedure analogous to that described in Example 37. (m.p. 257-260°C.) ($DMSO-d_6$) $\delta$ 1.32-1.56 (6H, m); 2.20-2.50 (4H, m); 3.28 (3H, s); 3.98-4.08 (1H, m); 4.30-4.44 (2H, m); 6.60-6.85 (3H, s); 7.40 (2H, broad s); 7.74 (1H, broad t).

EXAMPLE 40

3-[(7-(N-Piperidinylmethyl)-2,3-dihydro[1,4]benzodioxin-2-yl)-2-ethylamino]-4-methyl-5-amino-1,2,4,6-thiatriazine 1,1-dioxide

The title compound was obtained as a colourless solid (m.p. 219-223°C.) from 3-[(7-(N-piperidinylmethyl)-2,3-dihydro[1,4]benzodioxin-2-yl)-2-ethylamino]-4-methyl-5-[4-methylphenoxy]-1,2,4,6-thiatriazine 1,1-dioxide by a procedure analogous to that described in Example 37.

(DMSO-$d_6$) δ 1.32-1.55 (6H, m); 1.78-1.92 (2H, m); 2.25-2.45 (4H, m); 3.22 (3H, s); 3.86-3.96 (1H, m); 4.22-4.38 (2H, m); 6.74-6.88 (3H, m); 7.36 (2H, broad s); 7.56 (1H, broad s).

EXAMPLE 41

3-[(5-(N-Piperidinylmethyl)-2,3-dihydro[1,4]benzodioxin-2-yl)-2-ethylamino]-4-methyl-5-amino-1,2,4,6-thiatriazine 1,1-dioxide

The title compound was obtained from 3-[(5-(N-piperidinylmethyl)-2,3-dihydro[1,4]benzodioxin-2-yl)-2-ethylamino]-4-methyl-5-[4-methylphenoxy]-1,2,4,6-thiatriazine 1,1-dioxide by a procedure analogous to that described in Example 37.

(DMSO-$d_6$) δ 1.32-1.60 (6H, m); 1.80-1.95 (2H, m); 2.30-2.55 (4H, m); 3.26 (3H, s); 3.78-4.45 (3H, m); 6.78-6.95 (3H, m); 7.40 (2H, broad s), 7.60 (1H, broad s).

EXAMPLE 42

3-[(7-(N-Piperidinylmethyl)-2,3-dihydro[4H][1,4]benzoxazin-2-yl)-2-ethylamino]-4-methyl-5-amino-1,2,4,6-thiatriazine 1,1-dioxide

The title compound was obtained from 3-[(7-(N-piperidinylmethyl)-2,3-dihydro[4H][1,4]benzodioxin-2-yl)-2-ethylamino]-4-methyl-5-[4-methylphenoxy]-1,2,4,6-thiatriazine 1,1-dioxide by a procedure analogous to that described in Example 37.

($CD_3OD$) δ 1.55-1.70 (2H, m); 1.70-1.88 (4H, m); 1.88-2.00 (2H, m); 2.90-4.70 (11H, m); 4.02 (2H, s); 4.05-4.15 (1H, m); 6.62 and 6.78 (2H, ABq, J 9Hz), 6.84 (1H, s).

EXAMPLE 43

3-[(6-(N-Piperidinylmethyl)-2,3-dihydro[1,4]benzodioxin-2-yl)methylamino]-4-methyl-5-(2-propynyl)amino-1,2,4,6-thiatriazine 1,1-dioxide

2-Propynylamine (propargylamine) (1ml) was added in one portion to a solution of 3-[(6-(N-piperidinylmethyl)-2,3-dihydro[1,4]benzodioxin-2-yl)methylamino]-4-methyl-5-(4-methylphenoxy)-1,2,4,6-thiatriazine 1,1-dioxide (0.4ml) in a mixture of methanol (10ml) and chloroform (50ml) at ambient temperature. The resulting homogeneous solution was stirred for 72 hours, after which time TLC showed the reaction to be complete. The solvent was evaporated and the residue triturated with ether to afford 0.33g of the title compound as a colourless solid.

(DMSO-$d_6$) δ 1.35-1.55 (6H, m); 2.24-2.38 (4H, m); 3.20-3.60 (7H, m); 3.90-4.10 (4H,m); 4.35-4.45 (2H, m); 6.78-6.92 (3H, m); 7.80-8.20 (2H, broad s).

EXAMPLE 44

3-[(8-(N-Piperidinylmethyl)-2,3-dihydro[1,4]benzodioxin-2-yl)methylamino]-4-methyl-5-(2-propynyl)amino-1,2,4,6-thiatriazine 1,1-dioxide

The title compound was obtained from 3-[(6-(N-piperidinylmethyl)-2,3-dihydro[1,4]benzodioxin-2-yl)methylamino]-4-methyl-5-(4-methylphenoxy)-1,2,4,6-thiatriazine 1,1-dioxide by a procedure analogous to that described in Example 43.

(DMSO-$d_6$) δ 1.35-1.65 (6H, m); 2.60-2.75 (4H, m); 3.95-4.50 (6H, m); 6.65-7.04 (3H, m); 8.00 and 8.10 (2H, each broad s).

EXAMPLE 45

3-[(7-(N-Piperidinylmethyl)-2,3-dihydro[1,4]benzodioxin-2-

yl)-2-ethylamino]-4-methyl-5-(2-propynyl)amino-1,2,4,6-thiatriazine 1,1-dioxide

The title compound was obtained from 3-[(7-(N-piperidinylmethyl)-2,3-dihydro[1,4]benzodioxin-2-yl)-2-ethylamino]-4-methyl-5-(4-methylphenoxy)-1,2,4,6-thiatriazine 1,1-dioxide by a procedure analogous to that described in Example 43.

(DMSO-$d_6$) δ 1.30-1.56 (6H, m); 1.80-1.95 (2H, m); 2.20 (1H, s); 2.24-2.40 (4H, m); 3.86-4.40 (6H, m); 6.70-6.90 (3H, m); 7.65 (1H, broad s); 8.05 (1H, broad s).

EXAMPLE 46

3-[(5-(N-Piperidinylmethyl)-2,3-dihydro[1,4]benzodioxin-2-yl)-2-ethylamino]-4-methyl-5-(2-propynyl)amino-1,2,4,6-thiatriazine 1,1-dioxide

The title compound was obtained from 3-[(5-(N-piperidinylmethyl)-2,3-dihydro[1,4]benzodioxin-2-yl)ethylamino]-4-methyl-5-(4-methylphenoxy)-1,2,4,6-thiatriazine 1,1-dioxide by a procedure analogous to that described in Example 43.

(DMSO-$d_6$) δ 1.30-1.58 (6H, m); 1.80-1.94 (2H, m); 2.30-2.40 (4H, m); 3.25 (3H, s); 3.85-4.45 (5H, m); 6.76-6.92 (3H, m).

EXAMPLE 47

1-Methyl-3-amino-5-[(6-(N-piperidinylmethyl)-2,3-dihydro-[1,4]benzodioxin-2-yl)methylamino]-1,2,4-triazole

A mixture of N-cyano-1-methyl-2-(phenylmethylene)-hydrazine-carboximidothioc acid, methyl ester (GB 2 023 133B) (0.84g) and 2-(aminomethyl)-2,3-dihydro-6-(N-piperidinylmethyl)[1,4]benzodioxin (1.0g) was heated at 70°C. under vacuum for 3 hours. The oily residue was taken up in acetone (30ml), 2M HCl (10ml) was added and the resulting homogeneous solution was stirred at ambient temperature for 16 hours. The mixture was extracted with ether, the pH of the aqueous phase adjusted to 10 with 1N NaOH solution, and re-extracted with ethyl acetate. The

extract was dried ($MgSO_4$) and evaporated to afford 0.78g of the title compound as a colourless solid on trituration with ether.

($DMSO-d_6$) δ 1.30-1.55 (6H, m); 2.25-2.35 (4H, m); 2.35 (3H, s); 2.30-2.55 (4H, m); 3.88-4.08 (1H, m); 4.30-4.42 (2H, m); 4.90 (2H, s); 6.54 (1H, t, J 6Hz); 6.75-6.90 (3H, m).

EXAMPLE 48

1-Methyl-3-amino-5-[(8-(N-piperidinylmethyl)-2,3-dihydro-[1,4]benzodioxin-2-yl)methylamino]-1,2,4-triazole

The title compound was obtained from 2-(aminomethyl)-2,3-dihydro-8-(N-piperidinylmethyl)[1,4]benzodioxin by a procedure analogous to that described in Example 47.

($DMSO-d_6$) δ 1.30-1.55 (6H, m); 2.30-2.42 (4H, m); 2.32 (3H, s); 3.96-4.06 (1H, m); 4.28-4.45 (2H, m); 4.85 (2H, s); 6.50 (1H, t, J 6Hz); 6.75-6.92 (3H, m).

EXAMPLE 49

1-Methyl-3-amino-5-[(7-(N-piperidinylmethyl)-2,3-dihydro-[1,4]benzodioxin-2-yl)methylamino]-1,2,4-triazole

A solution of N-cyano-1-methyl-2-(phenylmethylene)-hydrazine-carboximidothioc acid, methyl ester (GB 2 023 133B) (0.82g) and 2-(aminomethyl)-2,3-dihydro-7-(N-piperidinylmethyl)[1,4]benzodioxin (0.93g) in acetonitrile (20ml) was heated under reflux for 72 hours. The solvent was evaporated and the residue chromatographed on a column of silica using chloroform/methanol mixtures as eluant to give a yellow oil (1.57g). This material was dissolved in acetone (10ml), 2M HCl (15ml) was added and the resulting mixture was stirred at ambient temperature for 3 hours. The mixture was washed with ethyl acetate, basified with sodium hydroxide, and re-extracted with ethyl acetate. The extract was dried ($MgSO_4$) and evaporated to afford 0.83g of the title compound as a yellow gum, which showed as a single spot on TLC using chloroform/methanol (3:1) as solvent.

δ 1.35-1.48 (2H,m); 1.48-1.60 (4H, m); 2.30-2.40 (4H, m); 3.35 (2H, s); 3.42 (3H, s); 3.45-3.75 (2H, m); 3.90-4.50 (6H, m); 6.78-6.86 (3H, m).

EXAMPLE 50

1-Methyl-3-amino-5-[(5-(N-piperidinylmethyl)-2,3-dihydro-[1,4]benzodioxin-2-yl)-2-ethylamino]-1,2,4-triazole. Hemioxalate salt

The title compound was obtained from 2-(2-aminoethyl)-2,3-dihydro-7-(N-piperidinylmethyl)[1,4]benzodioxin by a procedure analogous to that described in Example 49. ($D_2O$) δ 1.30-2.00 (8H, m); 2.85-3.02 (2H, m); 2.40 (3H, s); 3.40-3.50 (4H, m); 3.50-3.60 (2H, m); 4.00-4.42 (3H, m); 6.94 (3H, s).

EXAMPLE 51

1-Methyl-3-amino-5-[(7-(N-piperidinylmethyl)-2,3-dihydro-[1,4]benzodioxin-2-yl)-2-ethylamino]-1,2,4-triazole

The title compound was obtained from 2-(2-aminoethyl)-2,3-dihydro-7-(N-piperidinylmethyl)[1,4]benzodioxin by a procedure analogous to that described in Example 49. δ 1.36-1.50 (2H, m); 1.50-1.62 (4H, m); 1.80-2.10 (2H, m); 2.30-2.42 (4H, m); 3.36 (2H s); 3.42 (3H, s); 3.55-3.66 (2H, m); 3.85-4.35 (4H, m); 6.75-6.90 (3H, m). m/e 372 ($m^+$)

Claims:

1. A compound of formula I

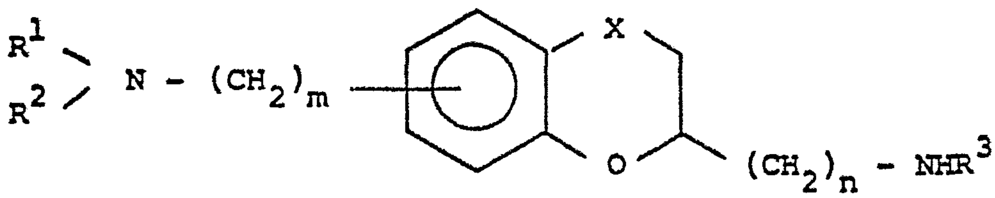

(I)

in which formula

$R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl group having from 1 to 4 carbon atoms as substituent,

X represents an oxygen atom or a group $NR^4$ in which $R^4$ represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substituents, which may be the same or different, for example, by one or two substitutents, especially by one substituent, selected from halogen atoms, straight and branched chain alkyl groups having from 1 to 4 carbon atoms, straight and branched chain alkoxy groups having from 1 to 4 carbon atoms, nitro and trifluoromethyl groups,

or $R^4$ represents a nitrogen protecting group;

m represents an integer of from 1 to 4;
n represents 1 or 2, especially 2; and
$R^3$ represents a group of formula II, III, IV, V or VI

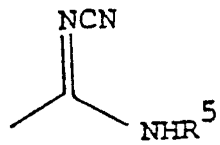
(II)

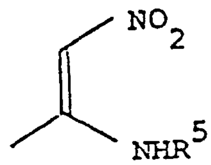
(III)

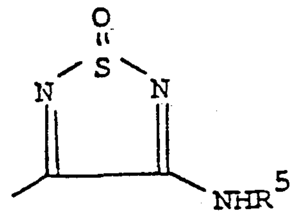
(IV)

(V)
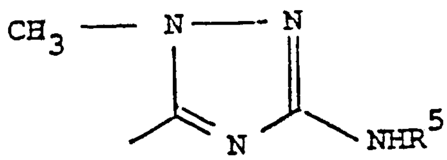

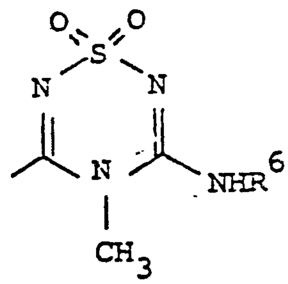
(VI)

in which formulae
$R^5$ represents a hydrogen atom or a straight or branched chain alkyl group having from 1 to 4 carbon atoms; and
$R^6$ represents a hydrogen atom or a straight or branched chain alkyl group having from 1 to 4 carbon atoms, a straight or branched chain alkenyl group having from 2 to 4 carbon atoms, or a straight or branched chain alkynyl group having 3 or 4 carbon atoms, or a salt thereof, especially a physiologically tolerable salt.

2. A compound as claimed in claim 1, wherein the group

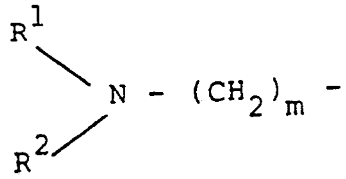

is in the 7 position.

3. A compound as claimed in claim 1 or claim 2, wherein when X represents an oxygen atom, $R^3$ represents a group of formula V, or represents a group of formula VI in which $R^6$ represents a hydrogen atom or a propargyl group.

4. A compound as claimed in claim 1 or claim 2, wherein X represents a group NH and $R^3$ represents a group of formula VI in which $R^6$ represents a hydrogen atom.

5. A compound as claimed in any one of claims 1 to 4, wherein $R^1$, $R^2$ and the nitrogen atom to which they are attached form a pyrrolidine, morpholine, N-methylpiperazine or piperidine ring.

6. A compound as claimed in any one of claims 1 to 5, wherein $R^1$, $R^2$ and the nitrogen atom to which they are attached form a piperidine ring, and (i) m is 1, X is oxygen, n is 2 and, preferably, $R^3$ represents a 3-amino-1-methyl-1,2,4-triazol-5-yl group, a 3-amino-4-methyl-1,2,4,6-thiatriazin-5-yl 1,1-dioxide group, or a 4-methyl-5-propargylamino-1,2,4,6-thiatriazin-3-yl 1,1-dioxide group, or (ii) m is 1, X is -NH-, n is 2 and, preferably, $R^3$ represents a 3-amino-4-methyl-1,2,4,6-thiatriazin-5-yl 1,1-dioxide group.

7. A process for the production of a compound of formula I as claimed in claim 1, which comprises (i) reacting a compound of formula VII

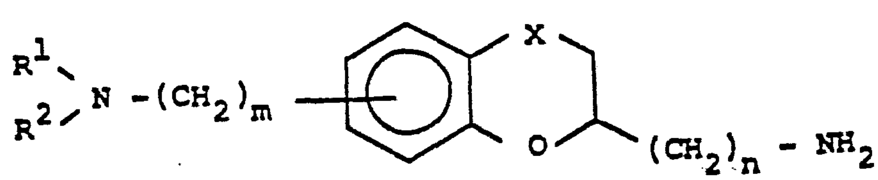

(VII)

in which $R^1$, $R^2$, X, m and n are as defined in claim 1 with a compound of formula IIa or IIIa

(IIa) 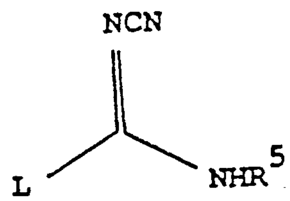

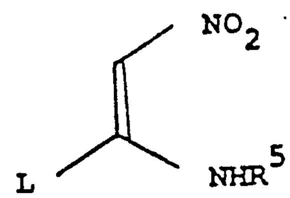 (IIIa)

in which L represents a leaving group, for example, a halogen atom or a group $-YR^7$ in which Y represents an oxygen or sulphur atom, a group SO or a group $SO_2$, and $R^7$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety, wherein a phenyl or phenalkyl group may be substituted by one or two substitutents selected from alkyl, alkoxy, methylenedioxy, phenoxy, halogen, dialkylaminoalkyl, trifluoromethyl, nitro, cyano, sulphonic acid, sulphonamide, amino, and mono- and di-alkylamino groups, an alkyl moiety having a straight or branched chain and from 1 to 4 carbon atoms; and $R^5$ is as defined in claim 1, to obtain a compound of formula I in which $R^3$ represents a group of formula II or III respectively; or

(ii) reacting a compound of formula VII as defined in (i) above with a compound of formula VIII

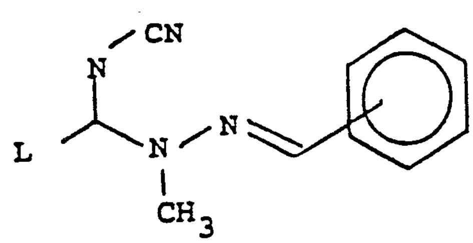 (VIII)

in which L is as defined in (i) above, and cyclising the resulting compound of formula IX

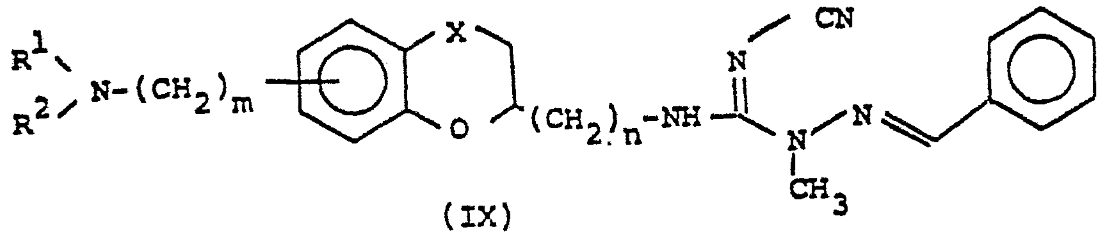

(IX)

to obtain a compound of formula I in which $R^3$ represents a group of formula V; or

(iii) reacting a compound of formula VII as defined in (i) above with a compound of formula IVa or VIa

(IVa) 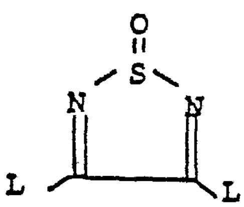

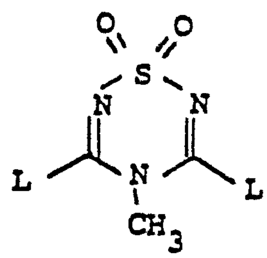 (VIa)

in which the two groups L each represents a leaving group as defined in (i) above, the two groups L being the same or different with the proviso that in the case of two leaving groups $-YR^7$ the two radicals $R^7$ can be the same or different, but the two groups Y must be the same, to give a compound of formula IVb or VIb respectively,

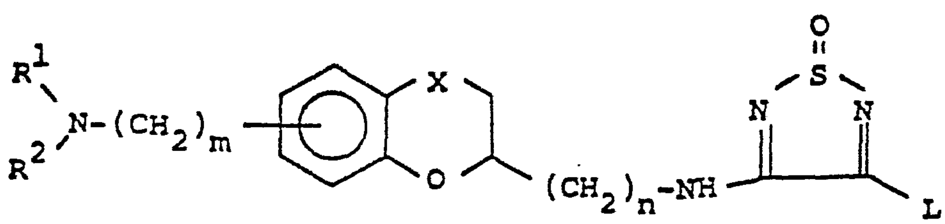 (IVb)

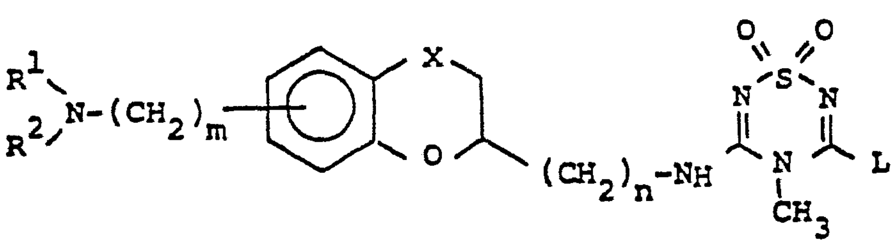 (VIb)

and reacting the resulting compound of formula IVb or VIb with a compound of formula Xa or Xb, respectively

(Xa) $H_2NR^5$ $H_2NR^6$ (Xb)

in which $R^5$ and $R^6$ are as defined in claim 1, to give a compound of formula I in which $R^3$ represents a group IV or VI, respectively.

8. A pharmaceutical preparation which comprises a compound of formula I as claimed in any one of claims 1 to 6 or a physiologically tolerable salt thereof as active ingredient, in admixture or conjunction with a pharmaceutically suitable carrier.

9. A compound of formula I as claimed in any one of claims 1 to 6 for use in the manufacture of a medicament, especially a medicament for use as a histamine H-2 antagonist.

Patent Claims for Austria:

0204148

1. Process for the production of a compound of formula I

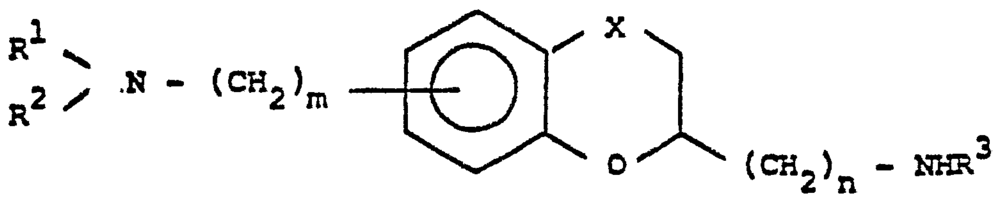

(I)

in which formula

$R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl group having from 1 to 4 carbon atoms as substituent,

X represents an oxygen atom or a group $NR^4$ in which $R^4$ represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substituents, which may be the same or different, for example, by one or two substitutents, especially by one substituent, selected from halogen atoms, straight and branched chain alkyl groups having from 1 to 4 carbon atoms, straight and branched chain alkoxy groups having from 1 to 4 carbon atoms, nitro and trifluoromethyl groups,

or $R^4$ represents a nitrogen protecting group;

m represents an integer of from 1 to 4;

n represents 1 or 2, especially 2; and

$R^3$ represents a group of formula II, III, IV, V or VI

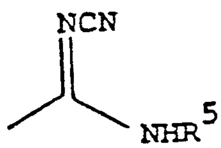

(II)

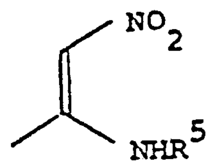

(III)

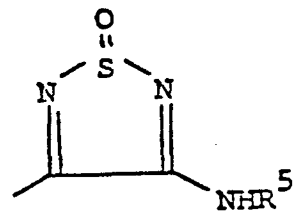

(IV)

(V)

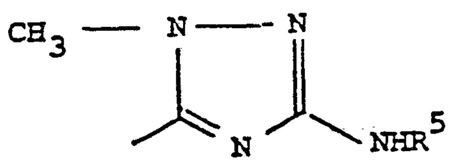

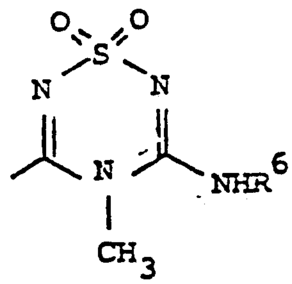

(VI)

in which formulae

$R^5$ represents a hydrogen atom or a straight or branched chain alkyl group having from 1 to 4 carbon atoms; and

$R^6$ represents a hydrogen atom or a straight or branched chain alkyl group having from 1 to 4 carbon atoms, a straight or branched chain alkenyl group having from 2 to 4 carbon atoms, or a straight or branched chain alkynyl group having 3 or 4 carbon atoms, or a salt thereof, especially a physiologically tolerable salt,

which comprises

(i) reacting a compound of formula VII

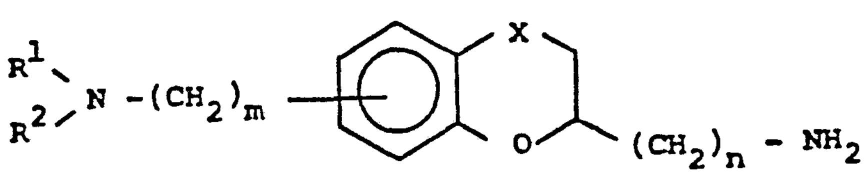

(VII)

in which $R^1$, $R^2$, X, m and n are as defined above with a compound of formula IIa or IIIa

(IIa) 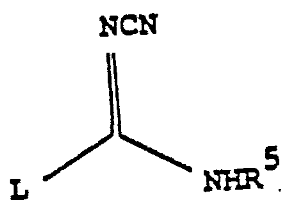 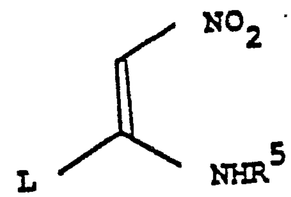 (IIIa)

in which L represents a leaving group, for example, a halogen atom or a group $-YR^7$ in which Y represents an oxygen or sulphur atom, a group SO or a group $SO_2$, and $R^7$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety, wherein a phenyl or phenalkyl group may be substituted by one or two substitutents selected from alkyl, alkoxy, methylenedioxy, phenoxy, halogen, dialkylaminoalkyl, trifluoromethyl, nitro, cyano, sulphonic acid, sulphonamide, amino, and mono- and di-alkylamino groups, an alkyl moiety having a straight or branched chain and from 1 to 4 carbon atoms; and $R^5$ is as defined in claim 1, to obtain a compound of formula I in which $R^3$ represents a group of formula II or III respectively; or

(ii) reacting a compound of formula VII as defined in (i) above with a compound of formula VIII

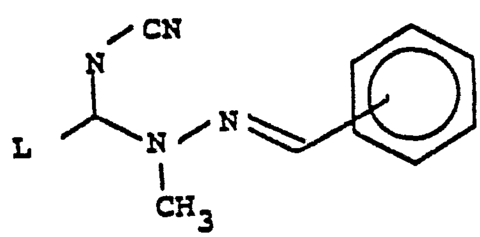 (VIII)

in which L is as defined in (i) above, and cyclising the resulting compound of formula IX

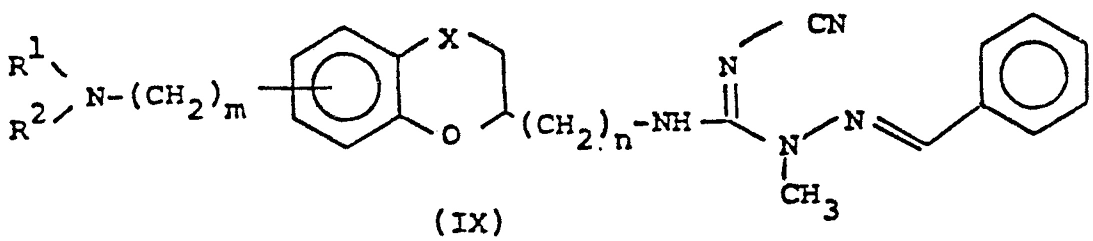

(IX)

to obtain a compound of formula I in which $R^3$ represents a group of formula V; or

(iii) reacting a compound of formula VII as defined in (i) above with a compound of formula IVa or VIa

(IVa)

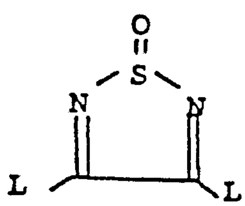

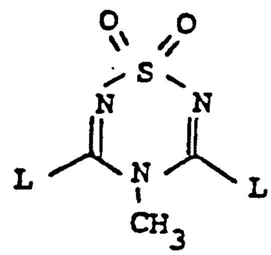

(VIa)

in which the two groups L each represents a leaving group as defined in (i) above, the two groups L being the same or different with the proviso that in the case of two leaving groups $-YR^7$ the two radicals $R^7$ can be the same or different, but the two groups Y must be the same, to give a compound of formula IVb or VIb respectively,

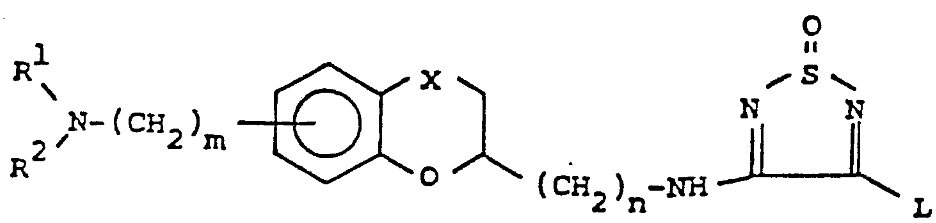

(IVb)

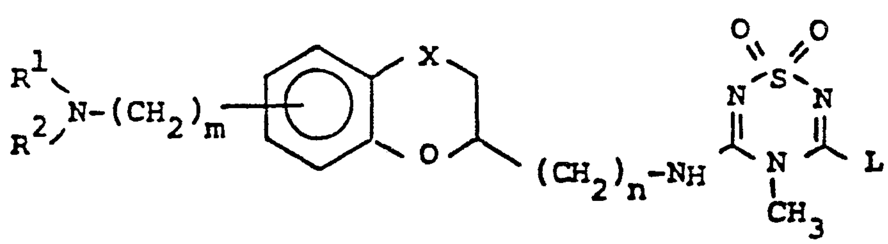

(VIb)

and reacting the resulting compound of formula IVb or VIb with a compound of formula Xa or Xb, respectively

(Xa) $H_2NR^5$ $H_2NR^6$ (Xb)

in which $R^5$ and $R^6$ are as defined in claim 1, to give a compound of formula I in which $R^3$ represents a group IV or VI, respectively.

2. A process as claimed in claim 1, wherein the group

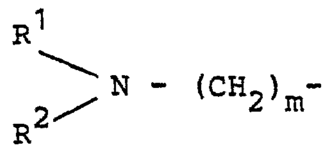

is in the 7 position.

3. A process as claimed in claim 1 or claim 2, wherein when X represents an oxygen atom, $R^3$ represents a group of formula V, or represents a group of formula VI in which $R^6$ represents a hydrogen atom or a propargyl group.

4. A process as claimed in claim 1 or claim 2, wherein X represents a group NH and $R^3$ represents a group of formula VI in which $R^6$ represents a hydrogen atom.

5. A process as claimed in any one of claims 1 to 4, wherein $R^1$, $R^2$ and the nitrogen atom to which they are attached form a pyrrolidine, morpholine, N-methylpiperazine or piperidine ring.

6. A process as claimed in any one of claims 1 to 5, wherein $R^1$, $R^2$ and the nitrogen atom to which they are attached form a piperidine ring, and (i) m is 1, X is oxygen, n is 2 and, preferably, $R^3$ represents a

3-amino-1-methyl-1,2,4-triazol-5-yl group, a 3-amino-4-methyl-1,2,4,6-thiatriazin-5-yl 1,1-dioxide group, or a 4-methyl-5-propargylamino-1,2,4,6-thiatriazin-3-yl 1,1-dioxide group, or (ii) m is 1, X is -NH-, n is 2 and, preferably, $R^3$ represents a 3-amino-4-methyl-1,2,4,6-thiatriazin-5-yl 1,1-dioxide group.

7. A process for the production of a pharmaceutical preparation which comprises admixing a compound of formula I as prepared according to any one of claims 1 to 6 or a physiologically tolerable salt thereof as active ingredient with a pharmaceutically suitable carrier.